# EUROPEAN PATENT SPECIFICATION

(11) **EP 0 439 535 B1**
(45) Date of publication and mention of the grant of the patent: **20.11.1996**
(21) Application number: 89912349.1
(22) Date of filing: 18.10.1989
(51) Int. Cl.: C12N 15/12, C07K 14/705, C07K 1/04

(54) **CDNAS CODING FOR THE GAMMA SUBUNIT OF THE HIGH-AFFINITY RECEPTOR FOR IMMUNOGLOBULIN E**
FÜR DIE GAMMA-UNTEREINHEIT DES REZEPTORS MIT HOHER AFFINITÄT FÜR IMMUNGLOBULIN E KODIERENDE CDNAS
ADNC CODANT POUR LA SOUS UNITE GAMMA DU RECEPTEUR DE GRANDE AFFINITE POUR L'IMMUNOGLOBULINE E

(30) Priority: 18.10.1988 US 259065
(43) Date of publication of application: 07.08.1991
(73) Proprietor: THE UNITED STATES OF AMERICA as represented by the Secretary UNITED STATES DEPARTMENT OF COMMERCE, Washington, DC 20231 (US)
(72) Inventor: KINET, Jean-Pierre, Bethesda, MD 20817 (US); METZGER, Henry, Chevy Chase, MD 20815 (US)
(74) Representative: Jump, Timothy John Simon
(86) International application number: US8904628
(87) International publication number: WO9004640

(56) References cited:
- PROCEEDINGS OF THE NATL. ACADEMY OF SCIENCES USA, vol. 85, September 1988; J.- P. KINET et al., pp. 6438-6487
- BIOCHEMISTRY, vol. 26, July 1987; J.-P. KINET et al., pp. 4605-4610
- BIOCHEMISTRY, vol. 26, May 1987; G. ALCARAZ et al., pp. 2569-2575

## Description

The present invention relates to the isolation and sequencing of the cDNA for the γ subunit of the high affinity receptor for immunoglobulin E (IgE) on mast cells and basophils. The invention is further directed to the expression of the receptor when the cDNA for its α, β, γ and subunits are simultaneously cotransfected.

The high affinity receptor for IgE is found exclusively on mast cells and basophils. This receptor, Fc_{ε}RI, plays a key role in allergy. When a multivalent allergen binds to the receptor-bound IgE, the consequent aggregation of the receptors leads to the release of mediators responsible for the allergic symptoms.

As may be seen from Table 1 below, Fc_{ε}RI is a tetrameric complex of non-covalently attached subunits: one IgE-binding α subunit, one β subunit and a dimer of disulfide-linked γ subunits (H. Metzger et al, Ann. Rev. Immunol. 4:419-470 (1986)).

**TABLE 1**

| Primary Structure of the High Affinity Receptor For IgE | | | |
|---|---|---|---|
| Subunit | No. per Receptor No. | Residues | Characteristics |
| α | 1 | 222 | Glycosylated; homology to Ig; binds IgE |
| β | 1 | 243 | Highly hydrophobic |
| γ | 2 | 62 | C terminally processed |

Complementary DNA (cDNA) for the α and the β subunits have recently been isolated (J.-P. Kinet et al, Biochemistry 26:4605-4610 (1987); A. Shimizu et al, Proc. Natl. Acad. Sci. USA 85:1907-1911 (1988); J. Kochan et al, Nucl. Acids Res. 16:3584 (1988)). However, previous to now there has not been disclosed the isolation and characterization of the γ subunit; nor has it been possible to express IgE-binding by transfected cells (J.-P. Kinet et al, Biochemistry 26:4605-4610 (1987); A. Shimizu et al, Proc. Natl. Acad. Sci. USA 85:1907-1911 (1988)).

The receptor with high affinity for IgE (Fc_{ε}RI) is found exclusively on mast cells, basophils and related cells. Aggregation of IgE occupied Fc_{ε}RI by antigen triggers both the release of preformed mediators such as histamine and serotonin, as well as stimulating the synthesis of leukotrienes. It is the release of these mediators which result in the allergic condition. The most thoroughly characterized Fc_{ε}RI is that of the rat basophilic leukemia (RBL) cell line. It consists of three different subunits: (1) A 40-50 Kilodalton (Kd) glycoprotein alpha chain which contains the binding site for IgE, (Z) A single 33 Kd beta chain and (3) Two 7-9 Kd disulfide linked gamma chains. The gene for human Fc_{ε}RI-has never been completely cloned and isolated. Only the gene coding for the alpha subunit of rat Fc_{ε} RI has been cloned and sequenced [see Kinet, et al., Biochemistry, 26:4605 (1987)].

Receptors that bind the Fc region of immunoglobulins ("Fc receptors") mediate their transport across membranes, stimulate a variety of cellular activities induced by antigen-antibody complexes, and may regulate the biosynthesis of antibodies. The cDNAs for several of the Fc receptors have been characterized. Three of the receptors (the receptor for polymeric immunoglobulins (1), the Fc receptors on macrophages and lymphocytes (2), and the high-affinity Fc, receptor on mast cells and basophils (3-5)] share a common feature: their immunoglobulin-binding portion contains two or more immunoglobulin-like domains.

The high-affinity Fc receptor is the only Fc receptor known to consist of multiple subunits. In addition to its immunoglobulin-binding α chain, it contains a β chain and two disulfide-linked γ chains (6). It has not yet been possible to express the cDNA for the α subunit on the surface of transfected cells (3,4). Possibly, as with other multisubunit receptors, one or more of the other subunits must be cosynthesized to achieve surface expression (7,8). The role of the β and γ subunits in the mechanism of action of this receptor is also of interest.

It is a general object of the present invention to isolate, characterize and clone the cDNA for the γ subunit of Fc_{ε}RI.

It is still another object of the invention to achieve the expression of Fc_{ε}RI when the cDNA for its α, β, and γ subunits are simultaneously cotransfected.

These, and other objects which will become clear to those skilled in the art from the following detailed description, have been accomplished by the isolation of a cDNA clone for the γ subunit and the successful expression of IgE receptor cDNAs in COS 7 cells. Surface binding of IgE on transfected COS 7 cells was found to require simultaneous co-transfection of cDNAs for all three subunits.

The instant invention also includes replicable prokaryotic or eukaryotic microbial expression vehicles capable of expressing the alpha subunit of the human Fc_{E}RI polypeptide in conjunction with beta and gamma subunits, transformed prokaryotic and eukaryotic microorganisms and cultures of these microorganisms which produce the alpha subunit of human Fc_{ε}RI polypeptide and beta and gamma subunits, as well as processes for producing the alpha subunit of the human Fc_{ε}RI polypeptide and beta and gamma subunits either through solid phase synthesis methods, or through the use of recombinant DNA technology in which the requisite gene sequences are inserted by means of a suitable DNA vector into a compatible prokaryotic or eukaryotic organism.

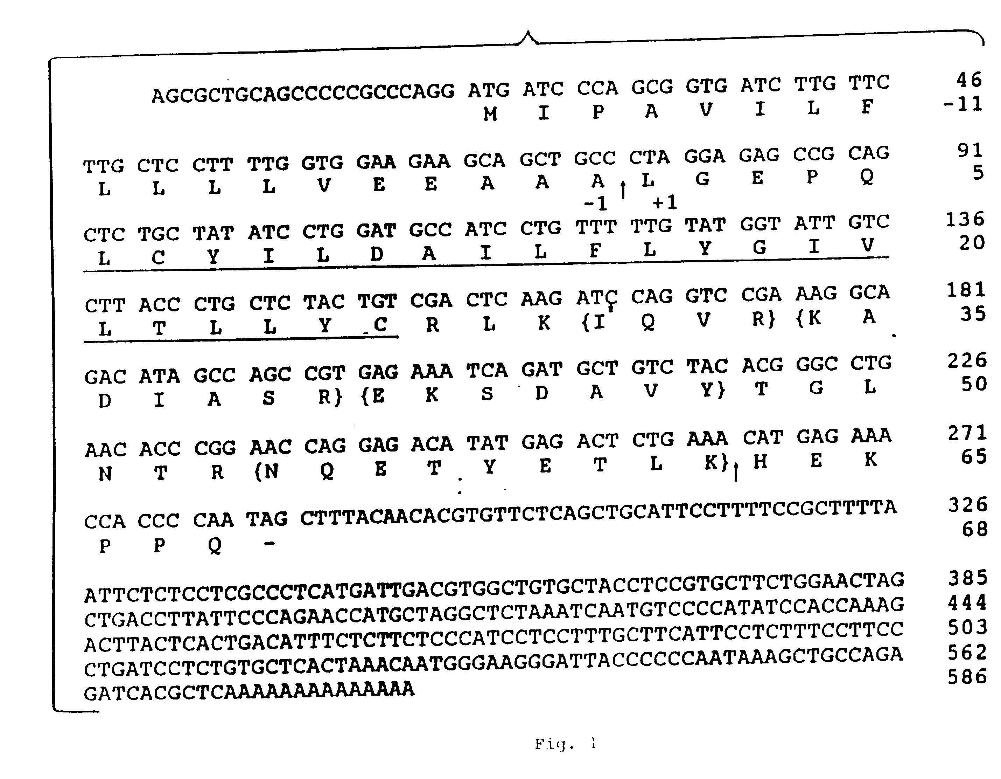

Figure 1 shows the nucleotide sequence of the γ subunit of rat Fc_{ε}RI and the amino acid sequence that it predicts. The putative transmembrane domain is underlined. Amino acid residues are numbered starting with the first residue of the mature protein. Residues 5' to residue 1 have negative numbers and include the residues encoding a putative signal peptide according to the criteria of G. von Heijne (Nucleic Acids Res. 14:4683-4690 (1986)). The N-terminal and C-terminal cleavage sites are indicated by an arrow. The four tryptic peptides which were covered and sequenced are bracketed. An asterisk denotes an ambiguous residue in the sequence of the first tryptic peptide.

Figures 2A-2C are hydropathicity plots of preicted sequences of Fc_{ε}RI: α subunit (panel A), β subunit (panel B) and γ subunit (panel C). The hydropathicity scale is according to Engelman et al (Ann. Rev. Biochys. Biophys. Chem. 15:321-353 (1986)). The summed hydropathicity values for the 20 amino acids in successive "windows" is plotted at the position corresponding to the tenth residue.

Figures 3A-3D show the formation of IgE rosettes by transfected COS 7 cells and RBL cells. COS 7 cells were cotransfected with the coding portions of α, β and γ cDNAS and sensitized with mouse IgE anti-DNP before being exposed to red cells derivatized with TNP (Panel A). As a positive control, RBL cells were similarly tested for rosette formation (Panel C). The specificity of the rosetting assay was assessed by preincubating the cotransfected COS 7 cells (Panel B) and RBL cells (Panel D) with rat IgE (which lacks the anti-DNP activity) prior to the addition of the mouse anti-DNP IgE.

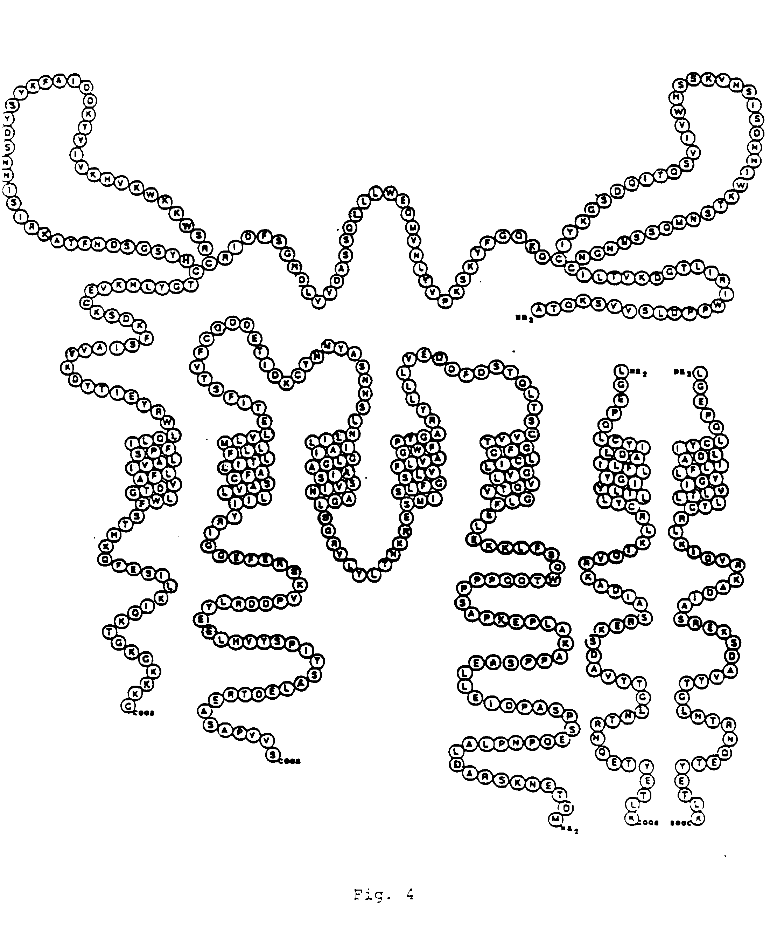

Figure 4 is a model of the tetrameric high affinity receptor for IgE. The polypeptides are shown in their fully processed form. The receptor is oriented such that the large extracellular portion of the α subunit is shown at the top and the remainder of the chain on the left. To the right of the α subunit is the β subunit with its four transmembrane segments and to the right of it, the dimer of γ chains. Cysteines 26 and 68 and cysteines 107 and 151 in the α chain are paired as they are likely to be disulfide linked, as are the homologous cysteines in the Fc γ receptors (M. Hibbs et al, J. Immunol. 140:544-550 (1988)). The putative transmembrane segments have all been shown as consisting of 21 residues and would be expected to be in an α-helical conformation. The single letter code for amino acids is used (M. Dayhoff et al, in Atlas of Protein Sequence and Structure, Suppl. 3, ed. M. Dayhoff, 363-373, Natl. Biomed. Res. Fndtn., Washington D.C. (1978)). Every 10th residue (starting from the N-terminus is shaded.

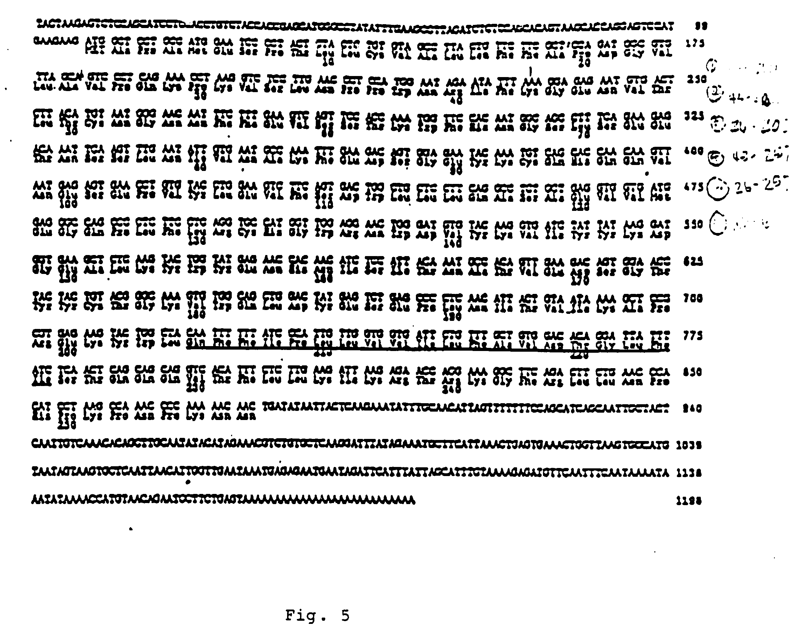

Figure 5 shows the nucleotide sequence and predicted amino acid sequence of human Fc_{ε}RI alpha cDNA.

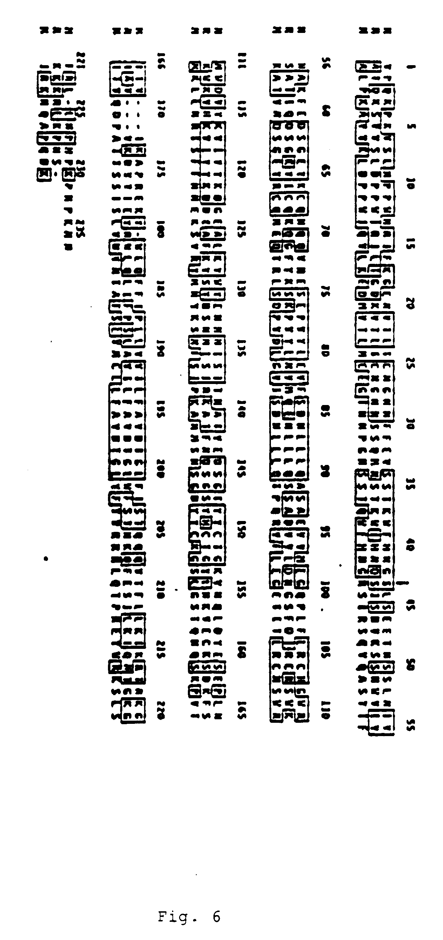

Figure 6 shows the amino acid sequence homology of rat Fc_{ε}RI alpha subunit (R), human Fc_{ε}RI alpha subunit (H), and mouse Fc_{ε}RI alpha subunit (M). The regions of identity between the three are boxed. The number one position corresponds to the site of the predicted mature N-terminus of each protein.

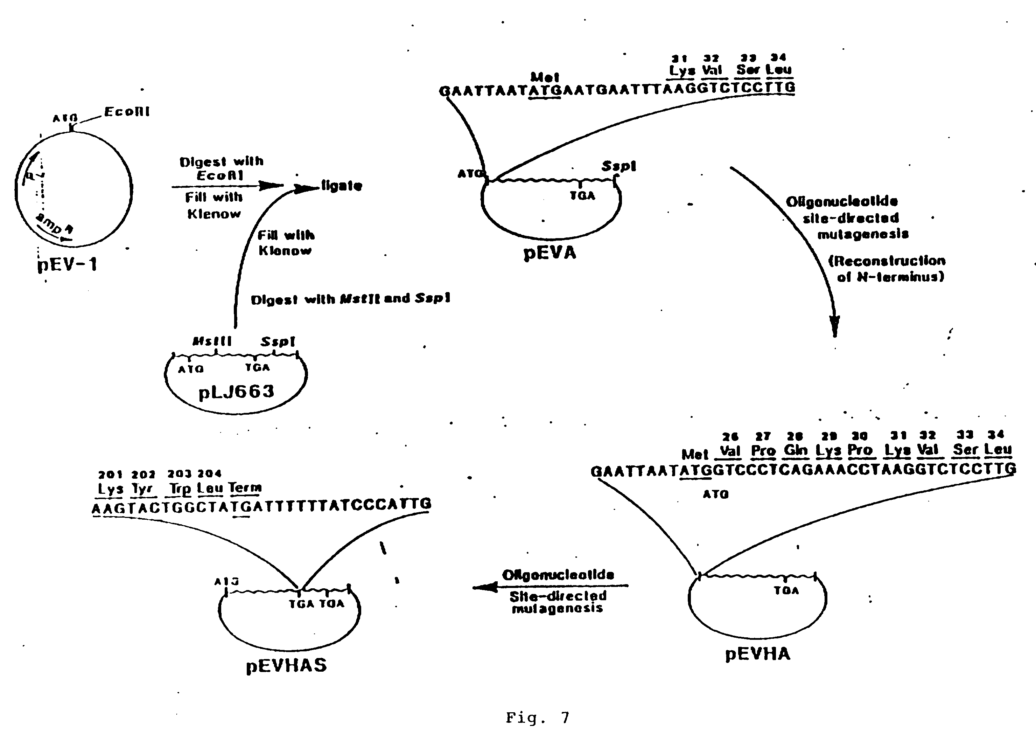

Figure 7 is a flow chart showing the construction of eukaryotic expression vectors which direct the synthesis of a complete biologically active Fc_{ε}RI alpha chain (pHAI, pHAII) or a soluble, secreted, biologically active Fc_{ε}RI alpha chain (pHASI, pHASII).

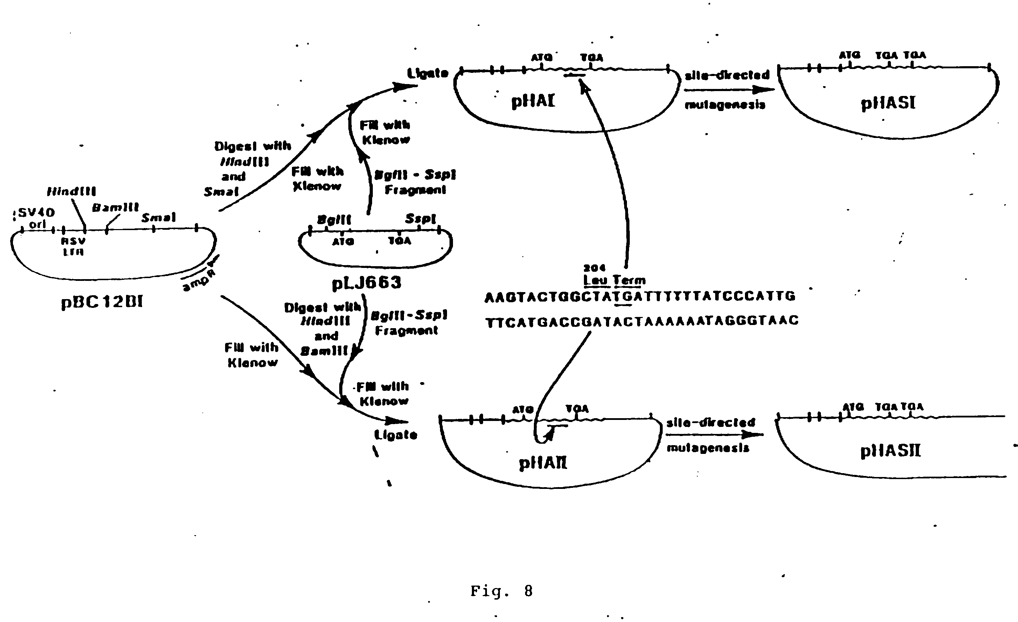

Figure 8 is a flow chart showing the construction of a prokaryotic expression vector which directs the synthesis of a soluble, biologically active FC_{ε}RI alpha chain (which consists of amino acid residues 26-204).

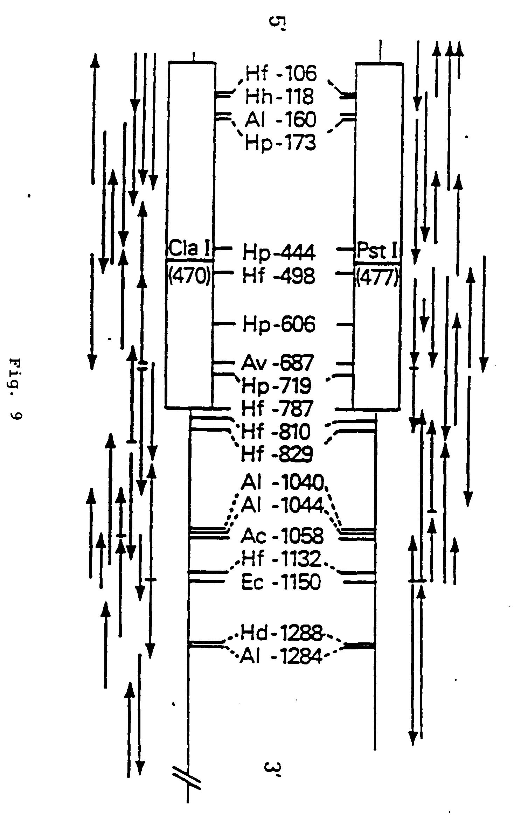

Figure 9 shows restriction maps for β cDNAs and strategy by which they were sequenced. The open rectangle indicates the sequence predicted to code for the β subunit; the lines indicate the 5' and 3' untranslated regions. The upper scheme shows the 1.5-kilobase (kb) clone containing a Pst I cleavage site. The lower scheme shows a 2.4-kb clone containing a Cia I cleavage site. The 3' region of the latter has been truncated as indicated by the slashes. Its untranslatd portion was sequenced as completely as the rest of the clone. Restriction sites are indicated by arrows: Hf. HindI:Hh, HhaI; Al Alu 1; Hp. Hpn. Av. Ava II:Ac.Acc1: Ec.EcoR1:Hd.HindIII. The horizontal arrows show the direction and extent of sequencing by the dideoxynucleotide chain-termination method.

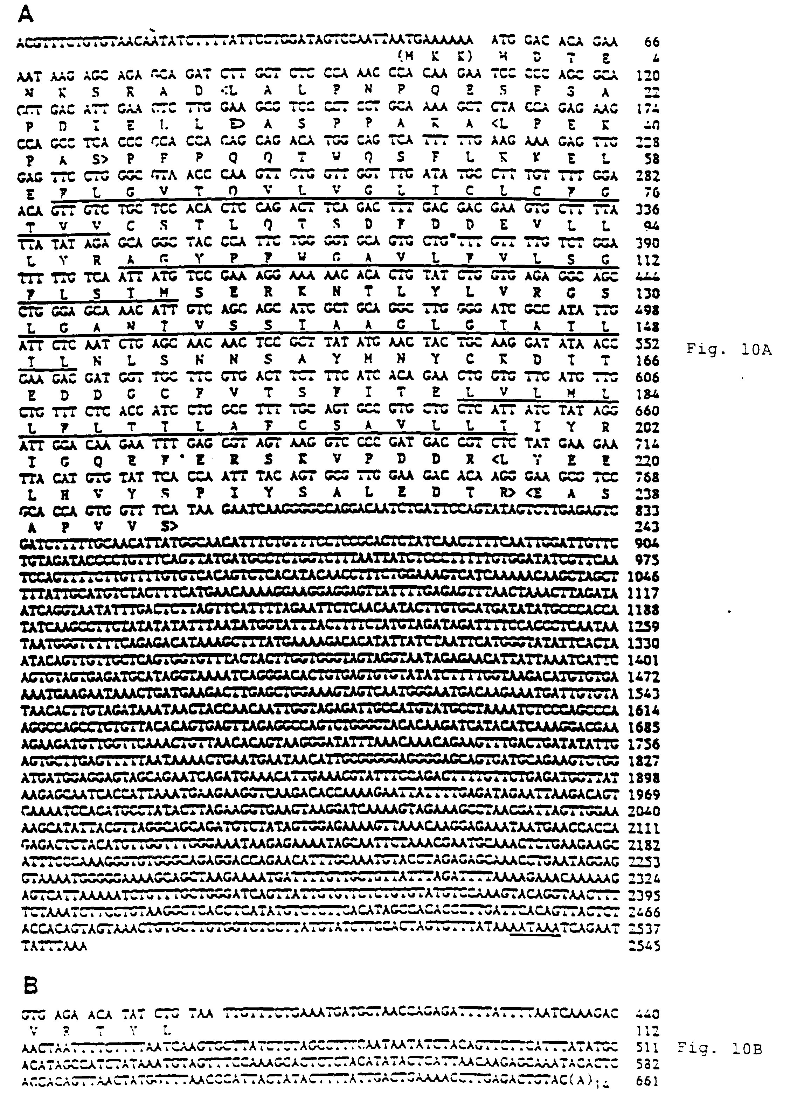

Figure 10A shows nucleotide and deduced amino acid sequences of the cDNA coding for the β subunit. Beginning at the arrowhead (▼), an alternative sequence (β) was observed in six clones. The putative transmembrane domains are underlined. The tryptic peptides of the subunit β from which the amino acid sequences were determined directly are bracketed (<>). A putative polyA signal near the end is underlined. Figure 10B shows a continuation of the nucleotide sequences of the deleted form of β CDNA. 3' to the junction indicated in A (▼).

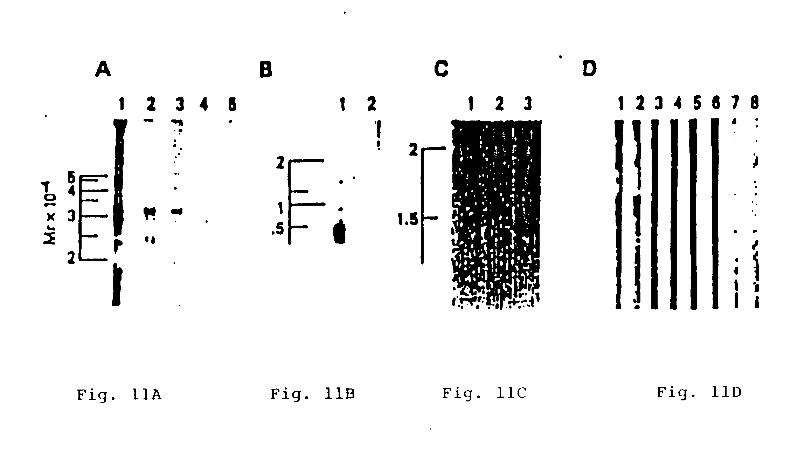

Figures 11A-11C show an expression of cDNA coding for the β subunit. Figure 11A is a comparison of in vivo and in vitro translation products. RBL cells were grown in [³⁵S]cysteine containing medium. The detergent extract of the cells was precipitated with mAb β (JRK) and, after vigorous washing, extracted with sample buffer and electrophoresed (lane 1). This experiment employed concentrations of detergent high enough to dissociate the receptor completely. A transcript from the β CDNA was treated in vitro in [³⁵S]methionine-containing medium (lanes 2, 3, and 5). A control incubation contained no cDNA (lane 4). The mixtures were allowed to react with monoclonal antibodies to β subunit after a cleaning immunoprecipitation. The specific washed precipitates were dissolved in sample buffer and electrophoresed; lanes 2 and 4. mAb β(HRK); lane 3, mAb β (NB1: lane 5 irrelevant monoclonal antibody [mAb(LB)]. An autoradiograph of the 12.5% polyacrylamide gel on which the specimens were analyzed under reducing conditions is shown. Figure 11B is a localization of one epitope to the NH_{α}-terminal peptides of the β subunit. A β cDNA-containing vector was digested with Hha 1 before transcription using T7 polymerase. The resulting mRNA was translated to generate an NH_{α} -terminal peptide of the β subunit (amino acid 1-21) labeled with [³⁵S]methionine. The mixture was allowed to react with mAb β (JRK) (lane 1) and the irrelevant mAb(LB) (lane 2). The precipitates were analyzed on a 17% gel under nonreducing conditions. Figure 11C is an expression by E. coli of a COOH-terminal fragment of the β subunit. A HindI fragment, containing nucleotides 499-787 was subcloned into an E. coli expression vector (16) and extracts were prepared. The proteins were electrophoresed as in A and transferred to nitrocellulose paper. The latter was allowed to react sequentially with monoclonal antibody mAb β (NB) developed with alkaline phosphatase-conjugated goat anti-mouse IgG (Fc) and developed in the usual way (14). An enlargement of the lower half ot the immunoblot is shown. Lane 1, extract from transformant without insert; lane 2 extract from transformant with insert in wrong direction; lane 3, extract from transformant with insert correctly oriented. Figure 11D shows reactivity of B subunits with polyclonal antibodies induced by E. coli-expressed HindI fragments. Purified IgE-receptor complexes were electrophoresed, transferred to nitrocellulose paper, and allowed to react with antibodies and subsequently with an appropriate alkaline phosphatase-conjugated anti-immunoglobulin antibody. Lane 1 mAb β (JRK); lane 2, mAb β (NB); lane 3, immune serum to fragment A; lane 5, immune serum to fragment B; lanes 1 and 6 preimmune sera corresponding to the immune sera in lanes 3 and 5, respectively; lanes 7 and 8, second antibody only. This gel was run without molecular weight standards.

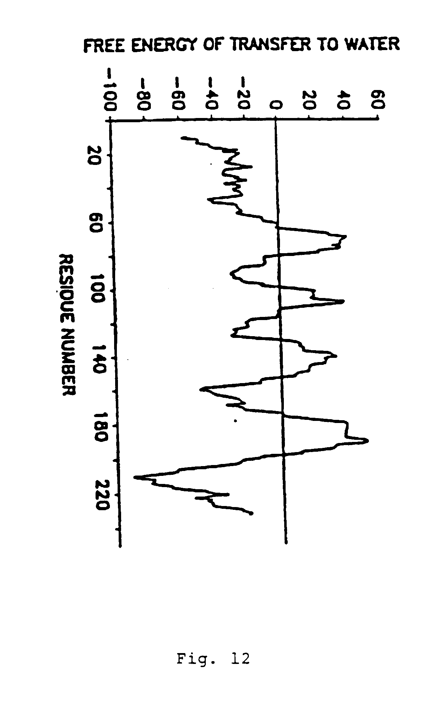

Figure 12 shows a hydropathicity plot of predicted sequence for the β subunit. The procedure and hydropathicity scale recommended by Engleman et al (21) was used. The net hydropathicity value for the 20 amino acids for each successive "window" is plotted at the position corresponding to the 10th residue. A net free energy of >20 kcal (1 cal = 4.18J) for transfer to water suggests a transmembrane segment (21).

The present invention is directed to the isolation, characterization and cloning of the cDNA for the γ subunit of the high affinity receptor for IgE (Fc_{ε}RI). Expression of the receptor on the surface of COS 7 cells is achieved by the present invention when the cDNA for all three subunits of Fc_{ε}RI are simultaneously cotransfected. This success in expression of IgE binding permits detailed analysis of the IgE-receptor interaction and thus enables the development of therapeutically effective inhibitors.

In order to isolate and characterize the cDNA for the γ subunit, cDNAs for the Fc_{ε}RI γ subunit were isolated from a λgt11 library prepared from rat basophilic leukemia (RBL) cells (J.-P. Kinet et al, Biochemistry 26:4605-4610 (1987)) using oligonucleotide probes. Four peptide sequences were identified in a tryptic digest of the Fc_{ε}RI γ subunits, and two of the peptides were used to synthesize two oligonucleotide probes (Figure 1). The library was screened in duplicate with these two probes and overlapping plaques identified. Three discrete plaques were purified, subcloned and found to contain similar inserts of 0.6 to 0.7 kilobases (kb).

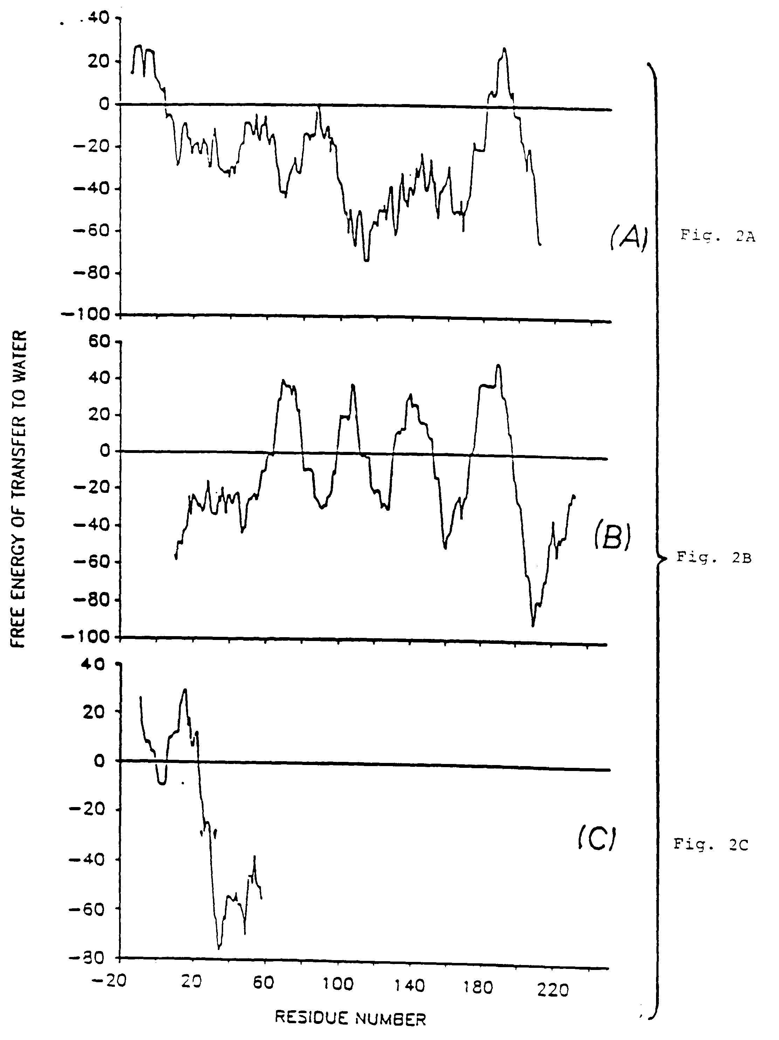

Figure 1 shows the complete nucleotide sequence of the γ cDNA, the deduced amino acid sequence and the position in the sequence of the four original tryptic peptides. Analysis of the sequence (Figure 2C) indicates an N-terminal hydrophobic signal peptide of 18 residues and a putative transmembrane domain separating a short extracellular portion of 5 residues from an intracytoplasmic domain. As predicted by earlier studies, the N-terminal processed γ subunit contains two cysteines, no methionine and no tryptophan residues (G. Alcaraz et al, Biochemistry 26:2569-2575 (1987)). Compositional analysis suggested that the γ subunit might contain one histidine residue (G. Alcaraz et al, Biochemistry 26:2569-2575 (1987)). However, recent biosynthetic dual labeling studies of the receptor using ³⁵S methionine and 3H histidine, clearly indicated that no trace of histidine was incorporated into the receptor-associated γ subunit. Since the open reading frame derived from three independent clones, each predicts a histidine six residues from the C-terminal end, it is expected that the γ subunit undergoes a C-terminal processing which clips off the histidine-containing segment. Furthermore, because the peptide immediately preceding this histidine was recovered (Figure 1), the C-terminal segment must be cleaved after Lys 63. The predicted molecular weight of the fully processed γ would therefore be 7139 Da, in close agreement with values obtained for the purified reduced γ on sodium dodecyl sulfate - urea gels (G. Alcaraz et al, Biochemistry 26:2569-2575 (1987)).

Polyclonal antipeptide antibodies to a heptamer and to a nonamer peptide of the γ subunit (Figure 1) were prepared and tested for reactivity with IgE-receptor complexes for RBL cells. Both purified antipeptide antibodies reacted in a Western blot assay with the unreduced dimer and the reduced monomer of partially purified γ subunits. In addition, both antibodies quantitatively precipitated receptor-bound ¹²⁵I-IgE, either from an extract of RBL cells or from a preparation of partially purified receptors. Taken together, these results leave no doubt that cDNAs isolated according to the present invention code for the γ subunit of Fc_{ε}RI.

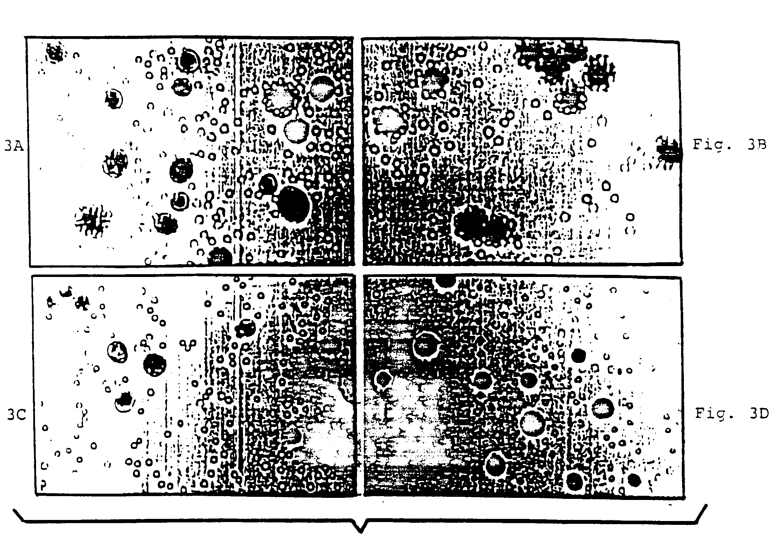

In order to achieve expression of the receptor on the surface of COS 7 cells, the coding region of the γ cDNA and of the previously isolated α and β cDNAs were first subcloned separately into the SV 40 promoter-driven expression vector psVL, prior to transfection into the COS-7 cells. IgE-binding was detected on the surface of the transfected cells by using an IgE rosetting assay (see Example 3). Figure 3A shows IgE-binding activity expressed by cells cotransfected with then α, β and γ subunits. Virtually all RBL cells, used as a positive control, formed rosettes (Figure 3C). The rosettes were completely inhibited by preincubation of the cells with rat IgE (Figures 3B and D) but not with human IgE (not shown). This coincides with the species specificity for the rat Fc_{ε}RI (A. Kulczycki et al, J. Exp. Med. 139:600-616 (1974)).

In order to study the requirements for surface expression of IgE-binding activity, the cells were transfected with different combinations of the cDNAs for the three subunits, as shown in Table 2 below.

**TABLE 2**

| Transfection Experiments | | | | |
|---|---|---|---|---|
| Cells Transfections | | | Expression | |
| | cDNA | No. | Receptor mRNA | IgE Binding (rosettes/cells counted) |
| COS 7 | 0 | 9 | 0 | 0 / 12,948 |
| | α | 2 | α | 0 / 4,050 |
| | αβ | 2 | αβ | 0 / 3,504 |
| | α | 4 | α | 0 / |
| 8,030 | | | | |
| | β | 1 | β | 0 / |
| 2,069 | | | | |
| | αβ | 29 | αβ | 920 / |
| 41,238 | | | | |
| | αβ | 4 | αβ | 0 / |
| 7,542* | | | | |
| RBL | 0 | - | αβ | |
| "100%" | | | | |

| | | | | |
|---|---|---|---|---|
| *Experiments where inhibiter was added (see Example 1). | | | | |

The above Table summarizes the data derived from all the transfection experiments performed by the present inventors to the time of filing the present application. The success rate of the transfection experiments has improved so that there is now routinely achieved 5±2% expression of IgE binding when α, β and γ are simultaneously cotransfected.

Successful transfection was achieved for all combinations, as assessed by Northern blotting, but rosette forming cells were only detected after cotransfection of the full set of the cDNAs. These results indicate that the β and γ subunits are required for surface-expression of the IgE-binding α subunit. It is further indicated that only the fully assembled receptor reaches the plasma membrane. This phenomenon has also been observed in other systems (M. McPhaul et al, Proc. Natl. Acad. Sci. USA 83:8863-8867 (1986); Y. Minami et al, Proc. Natl. Acad. Sci. USA 84:2688-2692 (1987)) and may be generally applicable to polymeric membrane proteins.

The easy dissociability of β and γ₂ from α (B. Rivnay et al, Biochemistry 21:6922-6927 (1982)) has raised persistent uncertainty about whether conceptually, γ₂ and β should be considered as subunits of Fc_{ε}RI or as "receptor associated" proteins. (An example of the latter is the CD3 complex which associates with the antigen receptor on thymus-derived lymphocytes (H. Clevers et al, Ann. Rev. Immunol. 6:629-662 (1988)). The subunit model for Fc_{ε}RI has been favored, for example, on the basis of the coordinate biosynthesis and catabolism of α, β and γ₂ (R. Quarto et al, Molec. Immunol. 22:1045-1052 (1985)). The new data on transfected cells obtained by the present invention provides the strongest evidence yet obtained that αβγ₂ is the minimal structure for Fc_{ε} RI.

The present model for the tetrameric Fc_{ε}RI receptor is illustrated in Figure 4. In this model each of the 589 amino acid residues of which the expressed receptor is composed is shown as a circle. In the diagram, the exterior of the cell would be at the top, the plasma membrane in which the receptor is embedded would be in the middle, and the interior of the cell towards the bottom. Each of the polypeptide chains (the α on the left, the β chain in the middle and the two γ chains on the right) contains one or more transmembrane segments.

The α chain is believed to contain two intra-chain disulfide loops, and the sequences of these loops show considerable homology with immunoglobulins (J.-P. Kinet et al, Biochemistry 26:4605 (1987); A. Shimizu et al, Proc. Natl. Acad. Sci. USA 85:1907 (1988); J. Kochan et al, Nucleic Acids Res. 16:3584 (1988)). Thus, the α subunit is another member of the immunoglobulin super-family (A. Williams et al, Ann. Rev. Immunol. 6:381 (1988)). The extracellular and transmembrane segments of the α chain show considerable homology with the immunoglobulin binding chain of Fc receptors that bind IgG (J. Ravetch et al, Science 234:178 (1986)) but the intracellular cytoplasmic tail is quite different. The carbohydrate residues that are covalently attached to the extracellular portion of the α chain are not indicated in Figure 4. There are seven potential sites for N-linked carbohydrates (J.-P. Kinet et al, Biochemistry 26:4605 (1987); A. Shimizu et al, Proc. Natl. Acad. Sci. USA 85:1907 (1988)), but which of these that are actually used by the cell remains to be determined. Studies show that the carbohydrate is not essential for the binding of IgE by this chain (B. Hempstead et al, J. Biol. Chem. 256:10717 (1981)).

The β chain contains four transmembrane segments (J.-P. Kinet et al, Proc. Natl. Acad. Sci. 85:6483 (1988)) and previous studies with monoclonal antibodies (J.-P. Kinet et al, Proc. Natl. Acad. Sci. 85:6483 (1988); J. Rivera et al, Mol. Immunol. 25:647 (1988)) show that the amino- and carboxyl-termini which are respectively 59 and 43 residues long, protrude from the cytoplasmic face of the plasma membrane. Similarly, the γ chains have an extensive intracellular extension but only very limited exposure to the exterior.

According to the present model, the putative transmembrane domains of the individual subunits are predicted from their respective hydropathicity plots (see Figure 2, wherein a net free energy of > 20 kcal/mol for transfer to water suggests a transmembrane segment or a leader peptide (D. Engelman et al, Ann. Rev. Biophys. Biophys. Chem. 15:321-353 (1986)). These plots suggest one, four and one hydrophobic domains for the α, β and each γ, respectively (i.e., seven transmembrane domains for the entire receptor). Members of a family of receptors interacting with G proteins also contain seven transmembrane domains (I. Herskowitz et al, Cell 50:995-996 (1987)). This family includes β and α adrenergic, muscarinic receptors and rhodopsin. Although no sequence homology between Fc_{ε}RI and these receptors is found, it is significant that an interaction between Fc_{ε}RI and G proteins has been postulated to explain at least some of the biochemical pathways activated by this receptor (S. Cockcroft et al, Nature 314:534-536 (1985)). The topology of the α and β subunits has been discussed in J.-P. Kinet et al, Biochemistry 26:4605-4610 (1987) and A. Shimizu et al, Proc. Natl. Acad. Sci. USA 85:1907-1911 (1988), in particular, the cytoplasmic localization of the C- and N- terminal portions of the β subunit. Two pieces of evidence support the topology of the γ-dimer as shown in Figure 4: The γ can be oxidatively iodinated on inverted vesicles but not on intact cells (D. Holowka et al, J. Biol. Chem. 259:3720-3728 (1984)) and, in vivo, γ becomes phosphorylated on threonine residues (R. Quarto et al, Mol. Immunol. 23:1215-1223 (1986)). None of the relevant residues are present in the small presumptive extracytoplasmic segment of γ but all are present on the presumptive cytoplasmic tail, i.e., two tyrosine and four threonine residues.

As a further means to examine the topology of the receptor, the putative extracellular and intracellular segments of the three subunits were analyzed for their relative content of basic residues, as.suggested by G. von Heijne Biochim. Biophys. Acta 947:307-333 (1988). He found the ratio of basic/total residues varies as a function of the length of the segment studied, but in general was substantially higher in the non-translocated (intracellular) segments than in the translocated (extracellular) segments of membrane proteins. Table 3 below shows a good correspondence between the ratios calculated for the present model and the ratios expected on the basis of "known" membrane proteins (G. von Heijne, Biochim. Biophys. Acta 947:307-333 (1988)), thereby providing independent support for the topological model presented here.

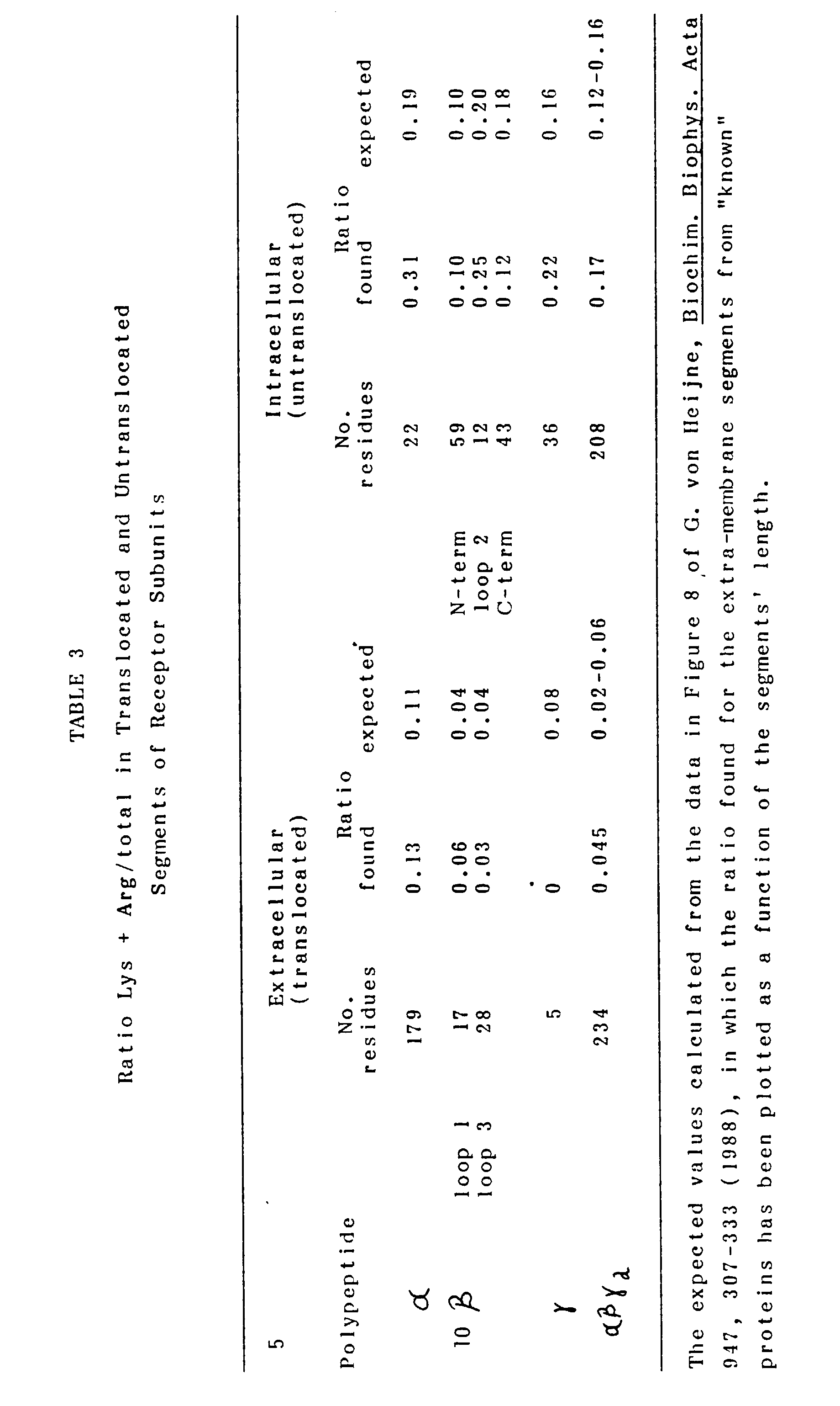

The present model clarifies several important features with respect to the organization of the subunits. The β and dimer of γ interact with each other; in detergent solutions they dissociate from the α as a unit before dissociating from each other (J. Rivera et al, Mol. Immunol. 25:647-661 (1988)), and occasionally, β and the γ dimer are observed to be disulfide-linked to each other (J.-P. Kinet, Biochemistry 22:5729-5732 (1983)). The likeliest candidates for this bond are γ-cys7 and β-cys80 which are predicted to be topologically close. This would then require that at least the γ-cys26 residues are disulfide-linked in the γ dimer. Preliminary data on the receptor biosynthesis suggest that α and β interact with each other.

The functional properties of Fc_{ε}RI are broadly similar to those of several Fc_{γ}R. Fc_{γ}R appears to bind to homologous segments of the immunoglobulin's Fc region (B. Helm et al, Nature 331:180-183 (1988); A. Duncan et al, Nature 332:563-564 (1988)), and the binding site on the receptor is found on a homologous polypeptide having immunoglobulin-like domains (J.-P. Kinet et al, Biochemistry 26:4605-4610 (1987); J. Ravetch et al, Science 234:718-725 (1986)). Both types of receptors need to be aggregated to initiate cell activation and, where studied, the latter appears to involve generation of broadly similar second messengers (H. Metzger et al, Ann. Rev. Immunol. 4:419-470 (1986); N. Hogg, Immunol. Today 9:185-187 (1988)). It is surprising, therefore, that whereas Fc_{ε}RI consists of four polypeptide chains, seven transmembrane segments and five cytoplasmic segments, Fc_{γ}Rs appear to perform similar functions with a much simpler structure, I.e., an α-like subunit alone. The extreme case is that of Fc_{γ}RIII which appears to lack even transmembrane and intracellular segments (P. Selvaray et al, Nature 333:565-567 (1988); D. Simmons et al, Nature 333:568-570 (1988); T. Huizinga et al, Nature 333:667-669 (1988)). It has been suggested that additional components of Fc_{γ} receptors may have thus far been missed. Possibly such components are even more easily lost upon solubilization of the receptors than are the β and γ subunits of Fc_{ε}RI (J.-P. Kinet et al, Biochemistry 24:4117-4124 (1985)). It seems reasonable to speculate that such hypothetical components would be homologous to β or γ, or both. The availability of genetic probes for the latter components will not permit an in-depth exploration of this possibility.

The success in expression of IgE binding achieved according to the present invention has important therapeutic implications. Degranulation of mast cells and basophils triggered by Fc_{ε}RI accounts for many of the symptoms of allergy. Given the high incidence of this disorder, the discovery of a specific inhibitor of IgE binding is expected to yield enormous therapeutic benefits. The development of such an inhibitor has been hampered by the lack of a practical in vitro assay for the binding of human IgE to the human receptors. For example, a recent assessment of IgE-derived peptides of their inhibitory capacity had to be determined by skin-testing (B. Helm et al, Nature 331:180-183 (1988)), a cumbersome and potentially dangerous procedure.

That the present invention achieves the expression of the transfected rodent receptor indicates that human Fc_{ε}RI can be similarly expressed. Alternatively, since at present only the cDNA coding for the human α subunit has been isolated (A. Shimizu et al, Proc. Natl. Acad. Sci. USA 85:1907-1911 (1988); J. Kochan et al, Nucl. Acids Res. 16:3584 (1988)), it is expected that it can be expressed in cotransfections with the cDNAs coding for the rodent β and γ chains.

A comparison between the human and rat α subunits is set forth in Table 4 below.

**TABLE 4**

| Comparative Properties of Human and Rat Alpha Chains | | | |
|---|---|---|---|
| | Species | | % Homology |
| Domain | Human | Rat | |
| Extracellular | 180 | 181 | 49 |
| Transmembrane | 21 | 21 | 67+ |
| Intracellular | 31 | 20 | 23 |
| Total | 232 | 222 | 47* |

| | | | |
|---|---|---|---|
| * Wt ave. | | | |
| + Human: WLQFFIPLLVVILFAVDTGLFISTQQQ Rat: WLQLIFPSLAVILFAVDTGLWFSTHKQ | | | |

It may be seen from the above Table that there is an overall homology between the human and rat alpha chains of about 47%, but an almost 70% homology in the presumed transmembrane domains. Indeed, when the transmembrane domains are examined closely, there is a stretch of 10 consecutive residues that are completely identical. This stretch of consecutive residues is underlined in the above Table.

Since the transmembrane segment is the region of the α chain that is most likely to interact with the β and γ chains, it was expected that the human α chain would be expressible, if transfected, along with the rat β and γ chains. This has proved to be the case as the present inventors have been able to express human IgE binding by COS cells transfected simultaneously with the human α and the rat β and γ subunits. It will be advantageous, of course, to have permanently transfected cell lines and for such lines, one will want to utilize the human β and γ subunits. The present inventors are in the process of identifying the coding sequences for these subunits so that preparing such transfectants will be straightforward. Thus, with the materials available now, it is already practical to search for peptide inhibitors of human IgE binding in vitro. To make the assay suitable for truly mass screening of drugs will require only minor extensions of the present work.

The genetic work, of course, provides much more than an assay, as important as the latter may be. Through directed mutation it will, in addition, allow the development of further information regarding the critical binding regions. It is expected that, using this information, rational drug design will become possible. It is further expected that it will be possible to block the function of the receptor itself, i.e., it will be possible to interfere with the early biochemical signals that result from activation of the receptor.

The present invention will be illustrated in detail in the following examples. These examples are included for illustrative purposes and should not be considered to limit the present invention.

### EXAMPLE 1

### Transfection Experiments

In the transfection experiments described above with reference to Table 2, COS-7 cells were transfected with different combinations of cDNAs for the three subunits of Fc RI (Figure 3). The rosetting assay was performed for each transfection shown in Table 2. The assessment of the mRNA by Northern blotting was performed one time only (on 2 x 10⁷ cells). Inhibitor was added to the cells in the experiments marked by an asterisk in Table 2 (50 g/ml of non-specific rat IgE was added to the cells 30 minutes prior to the addition of the specific mouse anti-DNP IgE).

### EXAMPLE 2

### Isolation and Characterization of the cDNA for the γ Subunit

Fc_{ε}RI was purified by affinity chromatography using TNP-lysine beads as described in G. Alcaraz et al, Biochemistry 26:2569-2575 (1987). The eluate was applied to sepharose 4B beads coupled by cyanogen bromide to monoclonal anti-β (JRK) (J. Rivera et al, Mol. Immunol. 25:647-661 (1988)). After washing the beads with 2 mM CHAPS in borate buffered saline at pH8, the bound material was eluted at 65°C with 0.1% sodium dodecyl sulfate, phosphate buffered saline, pH 6.5. The subunits from Fc_{ε}RI were then separated by HPLC size chromatography, the β and α containing fractions recovered, reduced, alkylated and digested with trypsin (J.-P. Kinet et al, Biochemistry 26:4605-4610 (1987)). The resulting peptides were separated by HPLC reverse phase chromatography as in J.-P. Kinet et al, Biochemistry 26:4605-4610 (1987). The chromatograms from the β and α digests were compared and the non-overlapping γ peptides were sequenced (J.-P. Kinet et al, Biochemistry 26:4605-4610 (1987)).

Oligonucleotide probes were synthesized according to the sequences of peptide 3 (residues 41 to 47) and of peptide 4 (residues 54 to 62). The sequences were GA(A/G)AA(A/G)TCIGA(T/C)GCTCTCTA and AA(T/C)CA(A/G)GA(A/G)ACITA(T/C)GA(A/G)ACI(T/C)TIAA. The methods used to screen the λgt11 library, to purify, subclone and sequence the positive clones are known in the art (J.-P. Kinet et al, Biochemistry 26:4605-4610 (1987)). Peptide 3 and peptide 4 were also synthesized using a peptide synthesizer ABI 431A. The purity of the synthetic peptides was assessed by HPLC reverse phase chromatography, amino acid composition and mass spectroscopy. The peptides were conjugated either to ovalbumin using m-Maleimidobenzoyl-N-hydroxysuccinimide ester (F.-T. Liu et al, Biochemistry 18:690-697 (1979)) at a molar ratio of 5:1 or to sepharose 4B with cyanogen bromide. Rabbits were immunized with the ovalbumin-conjugated peptides, the antisera collected and the antipeptide antibodies purified by affinity chromatography using sepharose 4B conjugated peptides. The antipeptide antibodies were tested for reactivity with the γ subunit of Fc_{ε}RI by Western blotting and for their ability to immunoprecipitate ¹²⁵I-IgE receptor complexes (J. Rivera et al, Mol. Immunol. 25:647-661 (1988)).

The nucleotide sequence of the γ subunit of rat Fc_{ε}RI obtained using the method of this invention, as well as the amino acid seauence that it predicts, are shown in Figure 1.

### EXAMPLE 3

### Formation of IgE Rosettes by Transfected COS 7 Cells and RBL Cells

The 810 bp EcoRI-Sty I restriction fragment of the α CDNA, the 965 bp EcoRI-EcoRV restriction fragment of the β cDNA and the 300 bp EcoRI-Dde I restriction fragment of the γ cDNA were subcloned separately into the Sma I site of the transient expression vector pSVL (Pharmacia, Uppsala, Sweden). These restriction fragments individually contained the entire coding sequence of the appropriate subunit and variable portions of untranslated sequences. The only foreign sequence was the starting EcoRI recognition sequence which belonged to the initial linker. Cultured COS 7 monkey kidney cells were then transfected with 40 µg of DNA by the standard calcium phosphate precipitation technique (L. Davis et al, in Basic Methods in Molecular Biology, ed. L. Davis, Elsevier, New York (1986)). After 48 hrs, the transfected cells (panels A and B of Figure 3), as well as RBL cells (panels C and D of Figure 3), were examined for surface expression of IgE binding by an IgE rosetting assay. The cells (5 x 10⁶ cells/ml) were incubated at room temperature with (panels B and D) or without (panels A and C) 50 µg/ml of non-specific rat IgE for 30 min and then with 5 µg/ml of anti-DNP-IgE (F.-T. Liu et al, J. Immunol. 124:2728-2736 (1980)). The cells were then rosetted with ox red blood cells that had been modified with 2,4,6-trinitrobenzene sulfonic acid according to a known method (M. Rittenberg et al, Proc. Soc. Exp. Biol. Med. 132:575-581 (1969)). The results are shown in Figure 3.

The DNA sequence which codes for the polypeptide corresponding to the alpha subunit of human Fc_{ε} RI is set forth in Figure 5. This DNA is elucidated by probing a human peripheral blood leukocyte cDNA library with the corresponding rat FcERI DNA according to methods well known to those skilled in the art. The cDNA obtained by hybridization was then subcloned using standard techniques. These cDNA inserts were mapped by restriction enzyme analysis and further subcloned and sequenced. The result was a DNA sequence of approximately 1,200 bases which coded for the human FcERI alpha subunit.

In the application of current recombinant DNA procedures, specific DNA sequences are inserted into an appropriate DNA vehicle, or vector, to form recombinant DNA molecules that can replicate in host cells. Circular double-stranded DNA molecules called plasmids are frequently used as vectors, and the preparation of such recombinant DNA forms entails the use of restriction endonuclease enzymes that can cleave DNA at specific base sequence sites. Once cuts have been made by a restriction enzyme in a plasmid and in the segment of foreign DNA that is to be inserted, the two DNA molecules may be covalently linked by an enzyme known as a ligase. General methods for the preparation of such recombinant DNA molecules have been described by Cohen et al. [U.S. patent No. 4,237,224], Collins et al. [U.S. patent No. 4,304,863] and Maniatis et al. [Molecular Cloning: A Laboratory Manual, 1982, Cold Spring Harbor Laboratory].

Once prepared, recombinant DNA molecules can be used to produce the product specified by the inserted gene sequence only if a number of conditions are met. Foremost is the requirement that the recombinant molecule be compatible with, and thus capable of autonomous replication in, the host cell. Much recent work has utilized Escherichia coli (E. coli) as a host organism because it is compatible with a wide range of recombinant plasmids. Depending upon the vector/host cell system used, the recombinant DNA molecule is introduced into the host by transformation, transduction or transfection.

Detection of the presence of recombinant plasmids in host cells may be conveniently achieved through the use of plasmid marker activities, such as antibiotic resistance. Thus, a host bearing a plasmid coding for the production of an ampicillin-degrading enzyme could be selected from unaltered cells by growing the host in a medium containing ampicillin. Further advantage may be taken of antibiotic resistance markers where a plasmid codes for a second antibiotic-degrading activity at a site where the selected restriction endonuclease makes its cut and the foreign gene sequence is inserted. Host cells containing properly recombinant plasmids will then be characterized by resistance to the first antibiotic but sensitivity to the second.

The mere insertion of a recombinant plasmid into a host cell and the isolation of the modified host will not in itself assure that significant amounts of the desired gene product will be produced. For this to occur, the foreign gene sequence must be fused in proper relationship to a signal region in the plasmid for DNA transcription called a promoter. Alternatively, the foreign DNA may carry with it its own promoter, as long as it is recognized by the host. Whatever its origin, the promoter is a DNA sequence that directs the binding of RNA polymerase and therefore "promotes" the transcription of DNA to messenger RNA (mRNA).

Given strong promotion that can provide large quantities of mRNA, the ultimate production of the desired gene product will be dependent upon the effectiveness of translation from mRNA to protein. This, in turn, is dependent upon the efficiency of ribosomal binding to the mRNA. In E. coli, the ribosome-binding site on mRNA includes an initiation codon (AUG) and an upstream Shine-Dalgarno (SD) sequence. This sequence, containing 3-9 nucleotides and located 3-11 nucleotides from the AUG codon, is complementary to the 3' end of E. coli 16S ribosomal RNA (rRNA) [Shine and Dalgarno, Nature 254:34 (1975)]. Apparently, ribosomal binding to mRNA is facilitated by base pairing between the SD sequence in the mRNA and the sequence at the 16S rRNA 3' end. For a review on maximizing gene expression, see Roberts and Lauer, Methods in Enzymology 68:473 (1979).

Most of the work in the recombinant DNA field to the present has focused on the use of bacterial expression systems such as E. coli. Yet, the use of bacterial cells has a number of undesirable aspects. For example, most proteins and polypeptides produced in E. coli accumulate in the periplasmic space. Recovery of these gene products thus requires disruption of the cells, a process which is inefficient and leads to a serious purification problem, as the desired product must be purified from the numerous other E. coli cellular constituents. Also, bacteria cannot carry out glycosylation which is needed to complete the synthesis of many interesting gene products or form the specific disulfide bonds which are essential for the proper conformation and biological activity of many eukaryotic proteins.

To overcome these deficiencies in bacterial expression systems, the attention of genetic engineers is increasingly turning to the use of eukaryotic host cells for recombinant DNA, not only to make desirable polypeptides and proteins but to study the control of gene expression as well. Cells such as yeast and mammalian cells can secrete desired gene products into the culture medium and can also carry out essential glycosylation processes. Yet, the use of mammalian cells for recombinant DNA cloning and expression also poses a host of technical obstacles that must be overcome. For example, the endogeneous plasmids that have proven to be so useful in bacteria are not replicated by higher eukaryotic cells. As a result, other approaches must be taken.

One approach has been to use the lower eukaryotic yeast, Saccharomyces cerevisiae, which can be grown and manipulated with the same ease as E. coli. Yeast cloning systems are available, and through the use of such systems the efficient expression in yeast of a human interferon gene has been achieved [Hitzeman et al., Nature (London) 293:717 (1981)]. Interferon genes do not contain introns, however, and it has been found that yeast cells do not correctly transcribe at least one heterologous mammalian gene that does contain introns, the rabbit β-globin gene (Beggs et al., Nature (London) 283:835 (1980)].

In another approach, foreign genes have been inserted into mammalian cells by means of direct uptake. This has been accomplished by calcium phosphate co-precipitation of cloned genes, by which procedure about 1-2% of the cells can generally be induced to take up the DNA. Such a low level of uptake, however, produces only a very low level of expression of the desired gene product. Where mammalian cells can be found which lack the thymidine kinase gene (tk⁻cells), better results can be obtained by co-transformation. Tk⁻cells, which cannot grow in selective HAT (hypoxanthine-aminopterin-thymidine) medium, can regain this lost enzymatic activity by taking up exogenous DNA (such as herpes simplex viral DNA) containing the tk gene through calcium phosphate co-precipitation. Other DNA covalently ligated to the tk DNA or merely mixed with it will also be taken up by the cells and will often be co-expressed [see Scangos et al., Gene 14:1 (1981)].

In a third approach, viral genomes have been used as vectors for the introduction of other genes into mammalian cells, and systems based upon Simian virus 40, papilloma-virus and adenovirus genomes have been described [see P.W.J. Rigby, Expression of Cloned Genes in Eukaryotic Cells Using Vector Systems Derived from Viral Replicants, in Genetic Engineering, vol. 3, R. Williamson, ed., Academic Press, New York, pp. 83-141 (1982) for a review]. These systems, however, suffer from the drawback of limited host cell range. Moreover, viral replication in these systems leads to host cell death. The use of retroviral DNA control elements avoids many of the disadvantages of these viral vector systems.

Gorman et al. [Proc. Natl. Acad. Sci. U.S.A. 79:6777 (1982)] have shown, for example, that the Rous sarcoma virus long terminal repeat (LTR) is a strong promoter that can be introduced into a variety of cells, including CV-1 monkey kidney cells, chicken embryo fibroblasts, Chinese hamster ovary cells, HeLa cells and mouse NIH/3T3 cells by DNA-mediated transfection.

The recombinant cDNA clone for human Fc_{ε}RI alpha chain was used to introduce these coding sequences into the appropriate eukaryotic expression vector in order to direct the synthesis of large amounts of the alpha chain polypeptide. In order for the alpha subunit to be expressed on eukaryotic cells it maybe necessary that the gene be complexed with that of the beta or gamma or other subunit. For expression of the secreted form this may not be necessary. Any of the appropriate eukaryotic expression vectors for example those set forth above, may be used. The expression of human Fc_{ε}RI alpha protein in eukaryotic cells will result in their synthesizing a mature IgE binding protein corresponding to human Fc_{ε}RI. The expression vectors may then be introduced into suitable eukaryotic cells by standard techniques. The synthesis of protein is monitored by demonstrating the ability of human IgE or rat IgE to bind to these cells.

The human Fc_{ε}RI alpha polypeptide may also be expressed in prokaryotic cells according to known methods. A recombinant cDNA clone for the human Fc_{ε}RI alpha chain is introduced into the appropriate prokaryotic expression vector to direct the synthesis of large amounts of IgE binding polypeptide derived from the alpha chain. This expression vector may then be transformed into suitable hosts and expression of a protein capable of binding to human IgE is then monitored.

Peptides corresponding to the complete or partial amino acid sequence of human Fc_{ε}RI alpha chain may also be synthesized by solid phase synthesis procedures for example, that generally described by Merrifield, Journal of the American Chemical Society 85, 2149 (1963). The peptide synthesized according to this method may be the entire alpha subunit or can be fragments which correspond to smaller, active portions of the alpha subunit.

The DNA sequences and polypeptides according to this invention exhibit a number of utilities including but not limited to:
1. Utilizing the polypeptide or a fragment thereof as an antagonist to prevent allergic response, or as a reagent in a drug screening assay.
2. Utilizing the polypeptide as a therapeutic.
3. Utilizing the polypeptide for monitoring IgE levels in patients.
4. Utilizing the DNA sequence to synthesize polypeptides which will be used for the above purposes.
5. Utilizing the DNA sequences to synthesize cDNA sequences to construct DNA probes useful in diagnostic assays.

### EXAMPLE 4

### Isolation of Human Fc_{ε}RI Alpha cDNA clones:

RNA was extracted from KU812 cells as described by Kishi, Leukemia Research, 9,381 (1985) by the guanidium isothiocyanate procedure of Chirgwin, et al., Biochemistry, 18,5294 (1979) and poly A+ RNA was isolated by oligo-dT chromatography according to the methods of Aviv, et al., P.N.A.S. U.S.A., 69,1408 (1972). cDNA synthesis was performed as previously described Kinet, et al., Biochemistry, 26,2569 (1987). The resulting cDNA molecules were ligated to EcoRI linkers, digested with the restriction enzyme EcoRI, size fractionated and ligated to λgt11 EcoRI arms as set forth in Young et al., Science, 222,778 (1983). The cDNA insert containing λgt11 DNA was packaged into bacteriophage lambda particles and amplified on Y1090. A total of 1.2x10⁶ independent cDNA clones were obtained. The cDNA library was plated onto Y1090 on 150 mm² plates (10⁵ per plate) and transferred to nitrocellular filters. The cDNA library filters were screened by in situ hybridization using a nick translated cDNA fragment as in Kochan, et al., Cell, 44,689 (1986). The cDNA fragment was obtained from the rat Fc_{ε}RI alpha cDNA corresponding to nucleotides 119-781. Positive plaques were identified, purified and the cDNA inserts were subcloned, using standard techniques, into the pGEM vectors (Promega Biotech, Madison, Wisconsin). The cDNA insert was mapped by restriction enzyme analysis, subcloned into derivatives of pGEM and sequenced using the dideoxynucleotide method of Sanger et al., P.N.A.S., 74,5463 (1977) following the GemSeq double strand DNA sequencing system protocol from Promega Biotech (Madison, Wisconsin). The DNA sequence was determined for both strands of the cDNA clone pLJ663 (nucleotides 1-1151) and for 300 bp of each end of clone pLJ 587 (nucleotides 658-1198). No discrepancy in DNA sequence between the two cDNA clones was observed.

The sequence for the human FceRI alpha cDNA is presented in Figure 5. The predicted amino acid sequence for the human Fc_{ε}RI alpha polypeptide is shown below the nucleotide sequence, beginning with methionine at nucleotide 107-109 and ending with asparagine at nucleotide 875-877. The site of the predicted mature N-terminus was determined to be valine at nucleotide 182-184 according to the rules set forth by von Heijne, Eur Journal of Biochem; 133,17; and Nucleic Acid Research, 14,4683 (1986). This predicts a 25 amino acid signal peptide. The rest of the cDNA sequence suggests that the human Fc_{ε}RI alpha chain contains a 179-residue extracellular portion (amino acid residues 26-204) with 2 homologous domains (14 out of 25 residues are identical; residues 80-104 and 163-190), a 20-residue transmembrane segment (residues 205-224) and a 33 residue cytoplasmic domain containing 8 basic amino acids. Overall, there is 49% identity between the human and rat Fc_{ε}RI alpha sequences, and 37% identity between the human Fc_{ε}RI alpha and mouse FcGR alpha (Figure 6). The greatest level of homology is within the transmembrane region where 9 amino acids surrounding the common aspartic acid residue are identical. 5

### EXAMPLE 5

### Expression of the Human Fc_{ε}RI Alpha Complete and Soluble Forms in Eukaryotic Cells

Using the recombinant cDNA clone for the human Fc_{ε}RI alpha chain, it is possible to introduce these coding sequences into an appropriate eukaryotic expression vector to direct the synthesis of large amounts of both a complete and soluble form of the alpha chain. For surface expression it may be necessary that the alpha subunit be complexed with the beta or gamma subunit whereas for the eukaryotic expression of the secreted form of the alpha subunit this may not be necessary. An appropriate vector for the purpose is pBC12BI which has previously been described in Cullen, (1987) Methods in Enzymology 152, Academic Press, 684. Construction of expression vectors coding for the complete alpha chain can be isolated as follows (Figure 7): A unique BgIII-SspI fragment (nucleotides 65-898) is isolated from pLJ663, the BgIII end is filled in with DNA polymerase I Klenow fragment and ligated into pBC12BI which has been restricted with either HindIII-BamHI or HindIII-SmaI (the ends are made blunt by filling in with DNA polymerase I Klenow fragment). The reason for attempting two different constructions is that the former contains a 3' intron while the latter does not. The presence or absence of introns may affect the levels of alpha protein which are synthesized in cells transfected by these vectors. Construction of expression vectors coding for the soluble form of the alpha chain would be accomplished by introducing a termination codon at nucleotides 719-721 of the coding region in the alpha chain of the expression vectors noted above (pHAI, pHAII, Figure 7). This would remove the putative transmembrane and cytoplasmic regions resulting in the synthesis of a secreted soluble form of the human alpha chain. Introduction of a termination codon is accomplished by oligonucleotide-directed site specific mutagenesis as outlined by Morinaga et al., Bio. Tech., 2,636 (1984). The sequence of the oligonucleotide will be 5' AAGTACTGGCTATGATTTTTTATCCCATTG 3'. The resulting expression vectors are pHASI and pHASII (Figure 7) and these will direct the synthesis of a truncated alpha protein corresponding to amino acids 1-204. Expression of this protein in eukaryotic cells will result in synthesis of a mature, IgE binding portion encompassing amino acid residues 26-204.

The expression vectors are then introduced into suitable eukaryotic cells such as CHO or COS by standard techniques such as those set forth in Cullen, (1987), Methods in Enzymology, 152, Academic Press, NY p. 684, in the presence of a selectable marker such as G418 or Methotrexate resistance. The selectable marker for Methotrexate resistance has an added advantage, since the levels of expression can be amplified by introducing the cells to higher levels of drugs. The synthesis of protein is monitored by demonstrating the ability of human IgE (or rat IgE) to bind to these cells (in the case of the complete alpha chain), or in the case of the soluble form of the alpha chain, to demonstrate that the protein secreted from these cells has the ability to bind IgE in the presence or absence of the beta.

### EXAMPLE 8

### Expression of the Human Fc_{ε}RI Alpha Soluble in Prokaryotic Cells

Using the recombinant cDNA clone for the human Fc_{ε}RI alpha chain, it is possible to introduce these coding sequences into an appropriate prokaryotic expression vector to direct the synthesis of large amounts of a soluble (non-membrane bound) IgE binding polypeptide derived from the alpha chain. An appropriate vector for this purpose is pEV-1 which has been described by Crowl, et al., Gene, 38,31 (1985). Construction of an expression vector coding for a soluble alpha chain can be isolated as set forth in Figure 8: a unique MstII-sSpI fragment (nucleotides 195-898) is isolated from pLJ663, the MstII end is filled in with DNA polymerase I Klenow fragment and ligated into PEV-1 which has been restricted with EcoRI, and the ends filled in with Klenow (Figure 8, pEVA). The N-terminus of the mature alpha chain is reconstructed by oligonucleotide directed-site specific mutagenesis. The sequence of the oligonucleotide will be 5' GAATTAATATGGTCCCTCAGAAACCTAAGGTCTCCTTG 3'. Introduction of this sequence into the expression vector pEVA aligns the Methionine residue of the EV-1 vector next to Valine-26 (the predicted mature N-terminus of the alpha chain) followed by amino acid residues 27-204 (pEVHA, Figure 8). Reconstruction of the soluble form FcERI alpha is accomplished by oligonucleotide site-directed mutagenesis. The sequence of the oligonucleotide will be 5' - AAGTACTGGCTATGATTTTTTATCCCATTG - 3' Introduction of this sequence into the expression vector, terminates polypeptide synthesis just prior to the start of the transmembrane region. The protein thus encoded by the expression vector pEVHAS, should faithfully direct the synthesis of a soluble form of the alpha chain, corresponding to amino acid residues 26-204.

This expression vector is then transformed into suitable hosts.

### EXAMPLE 9

### Isolation and Sequencing of β-subunit Peptides

Electroeluted β subunits from polyacrylamide gels were prepared as described (9). Tryptic peptides were separated by high-pressure liquid chromatography and sequenced as before (3).

### Cloning and Sequencing of cDNA

RNA extracted from rat basophilic leukemia (RBL)* cells by the guanidinium isothiocyanate method (10) was fractionated on an oligo(dT)-cellulose column (11) and used to construct a puc-9 and a λgt11 library (11,12). Colonies were screened as before (3) using oligonucleotides prepared on a model 380A automated DNA synthesizer (Applied Biosystems, Foster City, CA). cDNA inserts were subcloned into pGEM-4 or pGEM-3Z and the resulting double-stranded DNA was sequenced with the Gemseq/RT sequencing system according to the method recommended by the supplier (Promega Biotec. Madison, WI). Twenty-mer oligonucleotides corresponding to previously sequenced regions by this method, were used as primers to generate overlapping sequences otherwise difficult to obtain. In some instances, DNA sequencing was performed using Sequenase as recommended by the supplier (United States Biochemical, Cleveland).
* Abbreviation RBL, rat basophilic leukemia
The sequence reported herein is being deposited in the EMBL. GenBank data base UnteinGenesis, Mountain View, CA, and Eur. Mol. Biol. Lab., Heideiberg Accession No.1038-39.

In Vitro Transcription and Translation, cDNAs corresponding to the β subunit and various mutated or truncated forms thereof were subcloned into either pGEM-4 or pGEM-3Z transcription vectors (Promega Biotec). Unlabeled RNAs were synthesized using either SP6 or T7 polymerase as recommended by the supplier. Capping reactions were performed as reported (13). After digestion of the template with RNase-free DNase I, the RNAs were purified further by extraction with phenol/chloroform and three precipitations from ethanol. The RNA was then translated with a micro-coccal nuclease-treated lysate of rabbit reticulocytes in the presence of [³⁵S]methionine as recommended by the supplier (Promega Biotec). The products of translation were diluted 1: 1 with 20 mM detergent {3-[3- (cholamidopropyl)dimethylsam-monio]-I-propane sulfonate; in borate-buffered saline (pH 8) containing 30 µl of aprotinin per ml. 175 µg of phenylmethyl-sulfonyl fluoride per ml. 10 µg of leupeptin per ml. and 5 µ/g of pepstatin per ml and immunoprecipitated with monoclonal antibodies as described (14).

### Intrinsic Labelling of Receptors

Biosynthetic incorporation of labeled amino acids and monosaccharides was as described (15). The purification and analysis on gels and by immunoblotting of the IgE-receptor complexes have also been described (14).

### RNA Transfer Blotting

Thirty micrograms of total RNA was run on a 1% agarose gel containing 2% formaldehyde and blotted to nitrocellulose filters (11). The filters were hybridized with a restriction fragment of the β cDNA (nucleotides 1-174) as described (11) and washed with 15 mM (NaCl/1.5 mM sodium citrate at 65°C.

### Antibodies

Escherichia coli transformed with an expression vector containing the desired restriction fragments (16,17) were cultured and induced, and the fraction enriched for the recombinant protein was prepared as described (17). After separation on polyacrylamide gels in sodium dodecyl sulfate (NaDodSO₄) the transformant-specific protein was eluted and used to immunize rabbits. Approximately 100 µg of protein was injected in complete Freund's adjuvant; this was followed by a booster injection of 25 µg of protein in incomplete adjuvant. The isolation and characterization of monoclonal anti-β antibodies mAbβ (JRK) and mA (NB) the latter, a generous gift from David Halowka, Corneil University) have been described (14).

### Isolation of Peptides

Since repeated attempts to sequence intact β chains were unsuccessful, we isolated peptides from tryptic digests. A peptide (no. 1) isolated from an initial digest had the sequence Tyr-Glu-Glu-Leu-His-Vai-Tyr-Ser-Pro-Ile-Tyr-Ser-Ala-Leu-Glu-Asp-Thr. The same peptide from later digests showed an additional leucine at the NH₂ terminus and an arginine at the COOH terminus. The sequences of three other peptides, each isolated in substantial yields are indicated in a subsequent figure.

### Isolation of cDNA Clones

The initial sequence obtained for peptide 1 was used to construct two 26-mer oligonucleotides of 32-fold degeneracy: 
     A  G       AC
5'-GGIGA TA ACATGIA  TCATA-3'
     G  G       GT
 and 
        A  G       AC
5'-GGICT TA ACATGIA  TCATA-3'.
        G  C       GT
 A AGT11 library constructed from mRNA of RBL cells was screened with a 1:1 mixture of these oligonucleotides. Six positive clones gave similar restriction patterns. The clone containing the longest insert was sequenced according to the strategy shown in the upper portion of Fig.1. The sequence predicts possible starting codons at nucleotides 46-48 and 55-57, which would yield a polypeptide of 246 or 243 residues, respectively (Fig. 2A). The predicted M, of about 27,000 is some 20% less than the apparent molecular weight of β subunits when analzyed on polyacrylamide gels (18). In addition, no in-frame stop codon was apparent upstream of the start codon. To rule out the possibility that the true start codon was still further 5', we rescreened the cDNA library with a restriction fragment (nucleotides 7-474) and with a synthetic oligonucleotide probe (nucleotides 3-32). Twenty-eight additional clones were isolated and their restriction patterns were examined. Twenty were similar to the original clones. Only six additional nucleotides at the 5' end (nucleotides 1-6, Fig. 2A) were identified. Early termination was found in six clones, which otherwsie had the same sequence through nucleotide 375 (Fig. 2B). One 2.4-kb clone had cytidine 243 substituted with an adenine. This substitution abolishes the Pst I site and creates a new Cla I site at nucleotide 470. Also thereby, Ala-140 would become Asp-140 (Fig. 2A). Finally, one clone extended ≈350 base pairs (bp) in the 5' direction. The junction with the sequence shown in Fig. 2A was AATAAAACAAAAAAAAAAAAATG, the last two nucleotides of the newly generated ATG corresponding to nucleotides 8 and 9 of the previous sequence. It is likely that this clone simply resulted from the ligation of two independent cDNAs. Screening of the puc-9 library revealed three clones. However, the sequence of none of these extended 5' beyond nucleotide 84.

### RNA Transfer Blotting

RNA transfer blotting was performed under high stringency using a Pst I fragment probe (nucleotides I-474). RBL cells yielded two major bands at ≈ 2.7 kb and 1.75 kb, with the upper band having about twice the intensity of the lower one. A minor band at 1.2 kb was also noted. Negative results were obtained with a variety of cells that do not express high-affinity IgE receptors; the rat pituitary line GH3 (American Type Culture Collection no. CCL82.I), the rat glial cell line C6 (no. CCL107), the mouse Leydig cell line 1-10 (no. CCL83), and notably the mouse monocytic line J774 (no. TIB67) and the rat lymphoma "NTD" (14).

### In Vitro Expression

The β clone containing the Pst I site was transcribed in vitro with T7 RNA polymerase, and the resulting mRNA was translated with lysate of rabbit reticulocytes in the presence of [³⁵S]methionine. The unfractionated translated material showed a major component at M ≈32,000 compared to the control from which the RNA had been omitted or an alternative RNA (brome mosaic virus) had been substituted (data not shown). The monoclonal anti-β antibodies mAb β(JRK) and mAb β (NB)(14) (Fig. 3A, lanes 2 and 3)--but not an irrelevant antibody (lane 5)--precipitated radioactive material which on polyacrylamide gels in NaDodSO₄ showed a major band at M. 32,000. This band had the identical mobility as the upper band of the doublet precipitated by mAb β (JRX) from an extract of labeled RBL cells (lane 1). Although not seen well in the reproduction, the autoradiogram showed that the material synthesized in vitro also contained the lower molecular weight component seen in the in vivo synthesized β chains. The mobility of the in vitro synthesized protein was unaltered by reduction as has been previously observed with the β subunit. The clone containing the Cla I site (which lacks the first ATG codon) led to the synthesis of a protein whose mobility on gels was indistinguishable from that for the clone containing the Pst I site. On the other hand, an aberrant clone containing the newly generated ATG (above) induced the synthesis of a somewhat larger protein with an apparent M. of 33,500 (data not shown). In vitro translation of a transcript coding for the NH₂-terminal 21 amino acids of the β subunit led to a product precipitable by mAb β (JRK) (Fig. 3B).

### E. coli Expression

Two HindI fragments (A. nucleotides 106-98: B. nucleotides 499-787) were individually subcloned into an E. coli expression vector, and extracts were prepared from the induced cultures. The results of one immunoblotting experiment are shown in Fig. 3C. The material extracted from the bacteria transformed with a vector containing the HindI fragment B exhibited a M 14,000 component reactive with mAb β (NB) but not with mAb β (JRK) (Fig. 3C, line 3). The extract from the transformants containing the more NH₂-terminal HindI fragment A (residues 17-148) reacted with neither antibody (compare with above). Rabbit antibodies generated by fragment A reacted on immunoblots with purified receptors exactly at the position where the two monoclonal anti-β antibodies reacted (Fig. 3D, lanes 1-3) and quantitatively precipitated intact ¹²⁵I-labeled IgE-receptor complexes from unfractionated detergent extracts of RBL cells (data not shown).

### Biosynthetic Incorporation

By using biosynthetic incorporation of two different amino acids labeled distinguishably, we determined their ratio in the subunits of the receptor (Table 1, right part). The ratios of four distinctive amino acids to each other was in satisfactory agreement with the ratios predicted from the β cDNA for the β subunit predicts three potential glycosylation sites, we also performed a double-labeling experiment using [³H]mannose and [³⁵5]cysteine.

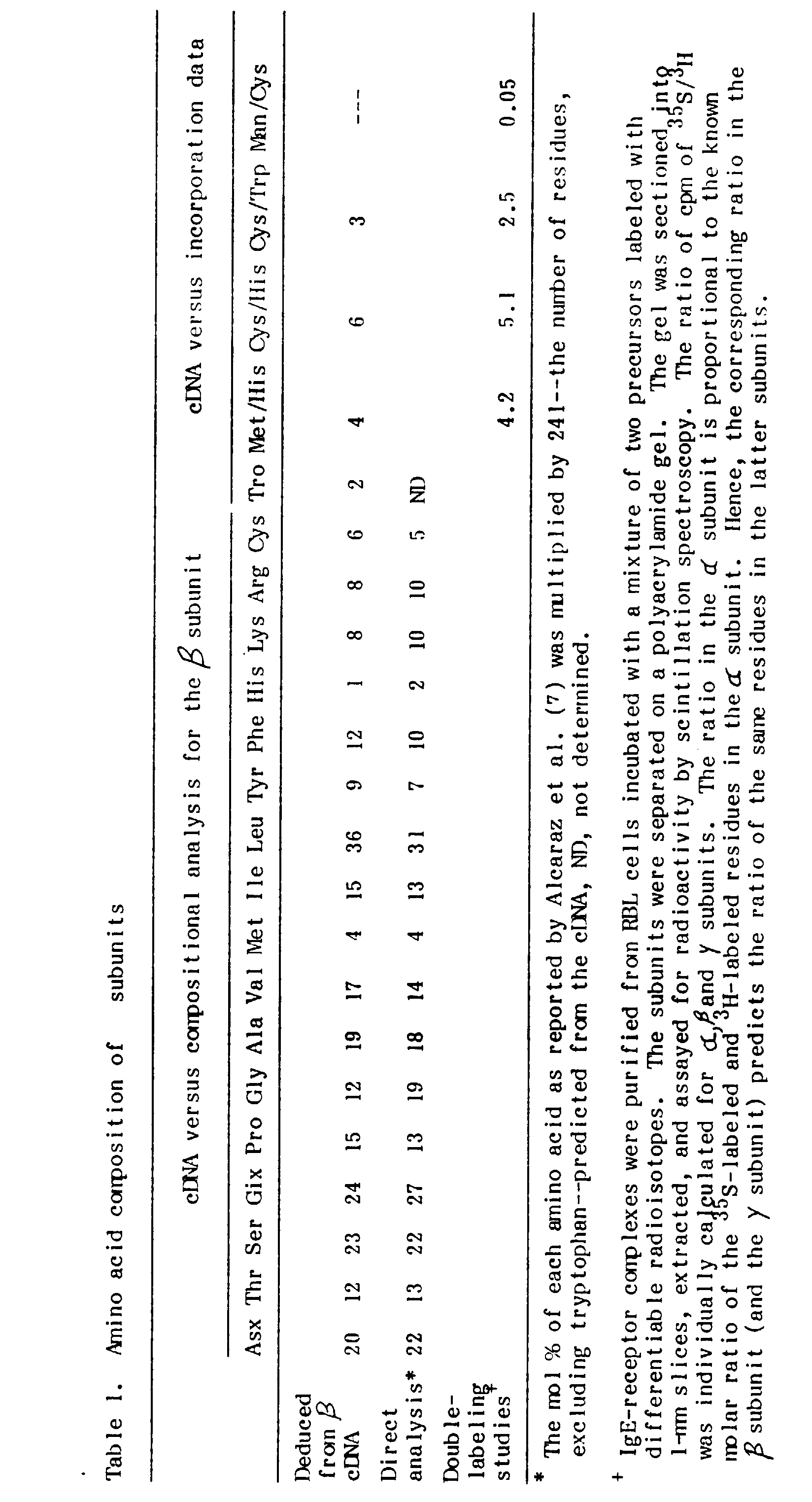

Based on the relative carbohydrate data reported for the α subunit (19) and correcting them on the basis of the peptide molecular weight for this chain predicted from the cDNA, we calculated that the α subunit contains ≈20 mol of mannose per mol. We are therefore able to determine the mannose/cysteine ratio in the β subunit from the double-labeling experiment. The results showed only 0.05 mol/mol of cysteine or 0.3 mol/mol of the β subunit (Table 1, right part, column 4).

### cDNA Codes for the β Subunit

There is ample evidence that the cDNAs we isolated code for the β subunit. (i) in vitro transcription of the cDNA and translation of the derived mRNA produce a protein whose apparent molecular weight on gel electrophoresis is indistinguishable from that of authentic β chains (Fig. 3). (ii) The cDNA accurately predicts the sequence of four peptides isolated from a tryptic digest of β chains (Fig. 2A) and a composition that agrees well with direct analyses and biosynthetic incorporations (Table 1). (iii) Two monoclonal antibodies reactive with discrete epitopes on the β subunit (I4) precipitate the protein synthesized in vitro from tne cloned cDNA (Fig. 3A), and one of them reacts with a fragment of the protein expressed in E. coli (Fig. 3B). (iv) Polyclonal antibodies raised against a fragment of the β subunit synthesized by E. coli transformants react with β chains on immunoblots (Fig. 3C) and with the IgE-receptor complex in solution.

### Initiation Site

The nucleotide sequence at the 5' end of the cloned cDNA (no. 1) does not in itself define the start of the open reading frame unambiguously. There is no leader sequence and no "in frame" stop codon preceding the presumptive start codon. In addition, the molecular weight deduced from the cDNA (M 27,000) is substantially lower than the one observed on NaDodSO₄ gels (M 32,000), although the subunit is not glycosylated. Therefore, it was possible that the start codon had been missed. Nevertheless, the aggregate data provide strong evidence that the full coding sequence for the β subunit has been recovered. (i) Extensive attempts failed to reveal cDNAs in either of two separate libraries with a more extended 5' sequence. (ii) The major species generated by 5' extension studies terminated precisely at the point at which most of our clones started. (iii) The second ATG codon at the 5' end meets the consensus characteristics of known initiation sites (20). That it is preceded by a nearby 5' ATG codon is uncommon, but not rare (20), and has been observed for the human α subunit (4,5). (iv) As already noted, in vitro transiation of an mRNA transcribed from the cDNA containing only the second ATG codon gives a polypeptide indistinguishable in length from the authentic β chains. An aberrant clone containing a start codon 48 nucleotides 5' to the presumed start codon directed the in vitro synthesis of a polypeptide with an apparent molecular weight appropriately greater than that of the β subunit (Results). Therefore, the correspondence in apparent molecular weight between authentic chains and the protein synthesized in vitro from clone 1 is meaningful. The RNA transfer blotting data show an mRNA of ≈2.7 kb, precisely what would be anticipated from the cDNA we have sequenced (Fig. 2), given a poly(A) tail of ≈200 nucleotides. In the discussion that follows we will assume that the β chain begins with the methionine residue coded for by the second ATG and is, therefore, 243 residues long.

### Alternative Forms of the β subunit

Only a single clone containing the Cla I restriction site was observed among the 37 clones analyzed. This clone likely resulted from a single base mutation during the cloning and is unlikely to represent a normally occurring mRNa. Conversely, six clones showing the deleted sequence (Fig. 2B) were observed and likely reflect an authentic species of mRNA. If translated, it would code for a Mᵣ 14,000 protein with only a single transmembrane segment.

### Sequence Characteristics

The sequence of the β subunit contains potential sites for N-linked glycosylation at residues 5.151. and 154. However, past and new incorporation data give no evidence for carbohydrate in the β subunit (refs. 15 and 18, and Table 1). The sequence shows no unusual features or homology to previously reported sequences, in particular to those associated with Fc receptors or with Fc binding factors.

### Topological Considerations

A hydropathicity analysis suggests that the β subunit crosses the plasma membrane four times (Fig. 4). The hydrophilic NH₄ and COOH terminus would therefore be on the same side of the membrane. Expression of fragments of the β cDNA indicate that mAb β-(NB) reacts within amino acid residues 149-243 (Fig. 3C) and that mAb β (JRK) reacts with a fragment containing residues 1-21 (Fig. 3B). Since neither antibody reacts appreciably with intact cells but both react strongly with cell sonicates, the combined results are consistent with the NH₂ and COOH terminus being studied on the cytoplasmic side of the plasma membrane.

Earlier studies had suggested that the β chain contained a M. 20,000 " "β1" domain resistant to proteolysis while membrane bound (13). This portion also contained those residues that were modified by an intrabilayer labeling reagent (18.22) and became linked to the α and/or y subunit when chemical crosslinking reagents were used (18) and to the y subunit when spontaneous disulfide linkage between the β and y₂ subunits occurred (23). The remainder, "β", appeared to contain the serine residues that became phosphorylated in situ (24,25) but has never been positively identified as a discrete fragment. The sequence predicted by the cDNA for the subunit suggests that part or all of either the NH₂-terminal 59 residues or the COOH-terminal 44 residues, or of both, is cleaved off to generate the β1 fragment.

### Cotransfection Experiments

The full-length coding sequences of the α and the β subunits were cotransfected in COS 7 cells by using a vector for transient expression. So far, no IgE-binding sites were expressed at the surface of transfected cells. Possibly all of the subunits will be necessary to achieve surface expression of the receptor.

### Subunits in Other Cells?

β Studies of the receptor with low affinity for IgE on macrophages revealed a component that could be chemically crosslinked to the IgE-binding portion and that had an apparent molecular weight similar to the β subunit of the high-affinity receptor (26). The peptides generated from this component by protease digestion appeared to differ from those released from β subunits, but it raised the possibility that other Fc receptors also contained β-like subunits that had heretofore escaped detection (see also ref. 14). So far, we have no evidence for this from RNA transfer blot experiments conducted at high stringency. In particular, J774 cells are known to contain Fc receptors whose immunoglobulin-binding chain shows considerable homology to the α chain of the high-affinity receptor for IgE (3). However, we could not detect mRNA for β chains by the methods we employed. Similarly, NTD lymphoma cells gave negative results even though they have Fc receptors and show a low molecular weight component that reacts with mAbβ (JRK) on immunoblots (14). We of course cannot exclude that Fc receptors have β -like subunits.
1. Mastov, K.E. Friedlander, M. & Blobel, G. (1984) Nature (London) 308, 37-43.
2. Ravetch, J.V., Luster, A.D., Weinshank, R. Kochan, J., Pavlavec, A. Portnoy, D.A., Hulmes, J., Pan, Y.-C. E. & Unkeless, J.C. (1986) Science 234, 718-725.
3. Kinet, J.-P. Metzger, H., Hakimi, J. & Kochan, J. (1987) Biochemistry 26, 4605-4610.
4. Shimizu, A., Tepler, I., Benfey, P.N., Berenstein, E.H., Siraganina, R.P. & Leder, P. (1988) Proc. Natl. Acad. Sci. USA 85, 1907-1911.
5. Kochan, J. Pettine, L. F., Hakimi. J., Kishi, K. & Kinet J.-P. (1988) Nucleic Acids Res. 16, 3584.
6. Metzger, H., Kinet, J.-P., Perez-Montfort, R. Rivnay, B. & Wank, S.A. (1983) in Progress in Immunology, eds. Yamamura, Y. & Tada T. (Academic, Orlando, F1), Vol. 5, pp. 493-501.
7. McPhaul M. & Berg, P. (1986) Proc. Natl. Acad. Sci. USA 83, 8863-8867.
8. Minami Y., Weissman, A.M. Samelson, L.E. & Klausner, R.D. (1987) Proc. Natl. Acad. Sci. USA 84, 2688-2692.
9. Alcaraz, G., Kinet, J.-P. Liu, T.-Y. & Metzer, H. (1987) Biochemistry 26, 2569-2575.
10. Chirgwin, J.M., Przybyia, A.E., MacDonald, R.I. & Rutter, W.J. (1979) Biochemistry 18, 5294-5299.
11. Maniatis, T., Fritsch, E.F. & Sambrook, J. (1982) Molecular Cloning: A Laboratory Manual (Cold Spring Harbor Lab., Cold Spring Harbor, NY).
12. Young, R.Y. & Davies, R.D. (1983) Proc. Natl. Acad. Sci. USA, 80, 1194-1198.
13. Contreras, R. Cheroutre, H., Degrave, W. & Fiers, W. (1982) Nucleic Acids Res. 10,6353-6362.
14. Rivera, J., Kinet, J.-P. Kim. J., Pucillo, C. & Metzger H. (1988) Mol. Immunol., in press.
15. Perez-Montfort, R. Kinet, J.-P. & Metzger, H. (1983) Biochemistry 27, 5722-5723.
16. Crowl, R., Seamans, C., Lomedico, P. & McAndrew, S. (1985) Gene 38, 31-38.
17. Portnoy, D.A. Erickson, A.H., Kochan, J., Ravetch, J.V. & Unkeless J.C. (1986) J. Biol. Chem., 261, 14697-14703.
18. Holowka, D. & Metzger, H. (1982) Mol. Immunol 19,219-227.
19. Kanellopoulos, J.M. Liu, T.Y., Poy, G. & Metzger, H. (1980) J. Biol. Chem., 255, 9060-0966.
20. Kozak, M. (1987) Nucleic Acids Res., 15.8125-8148.
21. Engleman, D.M. Steitz, T.A. & Goldman, A. (1986) Annu. Res. Biophys. Biophys. Chem. 15, 321-353.
22. Holowka, D., Gitler, C., Bercovici, T. & Metzger, H. (1982) Nature (London) 289, 806-808.
23. Kinet, J.-P., Perez-Montfort, R. & Metzger, H. (1983) Biochemistry 22, 5729-5732.
24. Perez-Montfort, R., Fewtrell, C. & Metzger, H. (1983) Biochemistry 22, 5733-5733.
25. Quarto, R. & Metzger, H. (1986) Mol. Immunol. 23, 1215-1223.
26. Finbloom, D. & Metzger, H. (1983) J. Immunol. 130, 1489-1491.

## Claims

1. A DNA segment coding for a polypeptide having the amino acid sequence of a chain of the γ-subunit dimer of Fc_{ε}RI or of a functionally equivalent homologue thereto.

2. A DNA segment as claimed in claim 1 having the nucleotide sequence shown in Figure 1.

3. A DNA segment as claimed in claims 1 or 2, wherein the polypeptide has the amino acid sequence shown in Figure 1.

4. A DNA segment as claimed in claim 3, wherein the polypeptide comprises the amino acid sequence from residue 1 to residue 62.

5. A DNA segment as claimed in claim 4, wherein the polypeptide comprises the animo acid sequence from residue 27 to residue 62.

6. A DNA segment as claimed in any one of the preceding claims, wherein the DNA segment is derived from a rat source.

7. A recombinant DNA molecule comprising a vector and a DNA segment as claimed in any one of claims 1-6.

8. A transformed organism comprising an organism transfected with a recombinant DNA molecule as claimed in claim 7.

9. A method for producing a recombinant polypeptide having the amino acid sequence of a chain of the γ-subunit dimer of Fc_{ε}RI or of a functionally equivalent homologue thereto, comprising culturing a transformed organism as claimed in claim 8 under conditions where said polypeptide is produced and isolating said polypeptide.

10. A method for producing a recombinant polypeptide complex having the amino acid sequence of the FC_{ε}RI high affinity receptor for IgE or of a functionally equivalent homologue thereto, comprising transfecting at least one cell with:
a recombinant DNA molecule coding for a polypeptide having the amino acid sequence of the α-subunit of Fc_{ε}RI or of a functionally equivalent homologue thereto;
a recombinant DNA molecule coding for a polypeptide having the amino acid sequence of the β-subunit of Fc_{ε}RI or of a functionally equivalent homologue thereto; and
a recombinant DNA molecule coding for a polypeptide having the amino acid sequence of a chain of the γ-subunit dimer of Fc_{ε} RI or of a functionally equivalent homologue thereto,
and culturing said transfected cell under conditions where said polypeptide complex is produced by said transfected cell and isolating said polypeptide.

11. A method for producing a recombinant polypeptide complex as claimed in claim 10, wherein the α-subunit-encoding recombinant DNA molecule is derived from a human source, and the β-subunit- and γ-subunit-encoding recombinant DNA molecules are each derived from a rat source.

12. A recombinant polypeptide complex having the amino acid sequence of the Fc_{ε}RI high affinity receptor for IgE or of a functionally equivalent homologue thereto obtainable by the method of claim 11.

13. A method of making a synthetic polypeptide having the amino acid sequence of a chain of the γ-subunit dimer or of a functionally equivalent homologue thereto, wherein said amino acid sequence is encoded by a DNA segment according to any one of claims 1-6 and said method comprises using a solid phase support as a synthesizing platform for making said synthetic polypeptide.

14. A method of making a synthetic polypeptide complex having the amino acid sequence of the Fc_{ε}RI high affinity receptor for IgE or of a functionally equivalent homologue thereto, said complex comprising an α-subunit, a β-subunit and a γ-subunit dimer, wherein the amino acid sequence of a chain of said γ-subunit dimer is encoded by a DNA segment according to any one of claims 1-6 and said method comprises using a solid phase support as a synthesizing platform for making said synthetic polypeptide.

## Patentansprüche

1. DNA-Segment, das ein Polipeptid mit der Aminosäuresequenz einer Kette des γ-Untereinheit-Dimers von-Fc_{ε}RI oder eines funktionell äquivalenten Homologs hierzu kodiert.

2. DNA-Segment nach Anspruch 1 mit der in Fig. 1 gezeigten Nucleotidsequenz.

3. DNA-Segment nach Anspruch 1 oder 2, wobei das Polipeptid die in Fig. 1 gezeigte Aminosäuresequenz aufweist.

4. DNA-Segment nach Anspruch 3, wobei das Polipeptid die Aminosäuresequenz vom Rest 1 bis zum Rest 62 aufweist.

5. DNA-Segment nach Anspruch 4, wobei das Polipeptid die Aminosäuresequenz vom Rest 27 bis zum Rest 62 aufweist.

6. DNA-Segment nach einem der vorhergehenden Ansprüche, wobei das DNA-Segment von einer-Ratten-Quelle abgeleitet ist.

7. Rekombinantes DNA-Molekül mit einem Vektor und einem DNA-Segment nach einem der Ansprüche 1 bis 6.

8. Transformierter Organismus, der einen mit einem rekombinanten DNA-Molekül nach Anspruch 7 transfektierten Organismus aufweist.

9. Verfahren zum Herstellen eines rekombinanten Polipeptids mit der Aminosäuresequenz einer Kette des γ-Untereinheit-Dimers von Fc_{ε} RI oder einem funktionell äquivalenten Homolog hiervon, welches das Kultivieren eines transformierten Organismus nach Anspruch 8 unter Bedingungen umfaßt, unter denen das Polipeptid produziert wird, und das Isolieren des Polypeptids umfaßt.

10. Verfahren zum Herstellen eines rekombinanten Polypeptidkomplexes mit der Aminosäuresequenz des Fc_{ε}RI-Rezeptors hoher Affinität für IgE oder eines funktionell äquivalenten Homologs hierzu, welches das Transfektieren mindestens einer Zelle umfaßt, die aufweist:
ein rekombinantes DNA-Molekül, das ein Polypeptid mit der Aminosäuresequenz der α -Untereinheit von Fc_{ε}RI oder eines funktionell äquivalenten Homologs hierzu kodiert,
ein rekombinantes DNA-Molekül, das ein Polypeptid mit der Aminosäuresequenz der β-Untereinheit von FC_{ε}RI oder eines funktionell äquivalenten Homologs hierzu kodiert, und
ein rekombinantes DNA-Molekül, das ein Polypeptid mit der Aminosäuresequenz der Kette des γ-Untereinheit-Dimers von Fc_{ε}RI oder eines funktionell äquivalenten Homologs hierzu kodiert,
und das Kultivieren der transfektierten Zelle unter Bedingungen, unter welchen der Polypeptidkomplex durch die transfektierte Zelle erzeugt wird, und das Isolieren des Polypeptids umfaßt.

11. Verfahren zum Herstellen eines rekombinanten Polypeptidkomplexes nach Anspruch 10, wobei das die α-Untereinheit kodierenden rekombinante DNA-Molekül von einer menschlichen Quelle abgeleitet wird und die die β-Untereinheit und die die γ-Untereinheit kodierenden rekombinanten DNA-Moleküle jeweils von einer Ratten-Quelle abgeleitet werden.

12. Rekombinanter Polypeptidkomplex mit der Aminosäuresequenz des Fc_{ε}RI-Rezeptors hoher Affinität für IgE oder eines funktionell äquivalenten Homologs hierzu, der durch das Verfahren nach Anspruch 11 erhältlich ist.

13. Verfahren zum Herstellen eines synthetischen Polypeptids mit der Aminosäuresequenz einer Kette des γ-Untereinheit-Dimers oder eines funktionell äquivalenten Homologs hierzu, wobei die Aminosäuresequenz durch ein DNA-Segment nach einem der Ansprüche 1 bis 6 kodiert wird und das Verfahren die Verwendung eines Festphasenträgers als Syntheseplattform zum Herstellen des synthetischen Peptids umfaßt.

14. Verfahren zum Herstellen eines synthetischen Polypeptidkomplexes mit der Aminosäuresequenz des Fc_{ε}RI-Rezeptors hoher Affinität für IgE oder eines funktionell äquivalenten Homologs hierzu, wobei der Komplex eine α-Untereinheit, eine β-Untereinheit und einen γ-Untereinheit-Dimer umfaßt, wobei die Aminosäuresequenz einer Kette des γ-Untereinheit-Dimers durch ein DNA-Segment nach einem der Ansprüche 1 bis 6 kodiert wird, und das Verfahren die Verwendung eines Festphasenträgers als Syntheseplattform zum Herstellen des synthetischen Polypeptids umfaßt.

## Revendications

1. Segment d'ADN codant pour un polypeptide ayant la séquence aminoacide d'une chaîne du dimère de la sous-unité γ de Fc_{ε}RI ou d'un homologue fonctionnellement équivalent.

2. Segment d'ADN selon la revendication 1 ayant la séquence de nucléotides décrite sur la figure 1.

3. Segment d'ADN selon la revendication 1 ou 2, dans lequel le polypeptide a la séquence d'aminoacides décrite sur la figure 1.

4. Segment d'ADN selon la revendication 3, dans lequel le polypeptide comprend la séquence d'aminoacides du résidu 1 au résidu 62.

5. Segment d'ADN selon la revendication 4, dans lequel le polypeptide comprend la séquence d'aminoacides du résidu 27 au résidu 62.

6. Segment d'ADN selon l'une quelconque des revendications précédentes, dans lequel le segment d'ADN provient du rat.

7. Molécule d'ADN recombinant comprenant un vecteur et un segment d'ADN selon l'une quelconque des revendications 1 à 6.

8. Organisme transformé comprenant un organisme transfecté avec une molécule d'ADN recombinant selon la revendication 7.

9. Procédé d'obtention d'un polypeptide recombinant ayant la séquence d'aminoacides d'une chaîne du dimère de la sous-unité γ de Fc_{ε}RI ou d'un homologue fonctionnellement équivalent, comprenant l'étape consistant à cultiver un organisme transformé selon la revendication 8 dans des conditions où l'on produit ledit polypeptide et à isoler ledit polypeptide.

10. Procédé d'obtention d'un complexe de polypeptide recombinant ayant la séquence d'aminoacides du récepteur à forte affinité Fc_{ε}RI pour IgE ou d'un homologue fonctionnellement équivalent, comprenant l'étape consistant à transfecter au moins une cellule avec :
une molécule d'ADN recombinant codant pour un polypeptide ayant la séquence d'aminoacides de la sous-unité α de Fc_{ε}RI ou d'un homologue fonctionnellement équivalent;
une molécule d'ADN recombinant codant pour un polypeptide ayant la séquence aminoacides de la sous-unité β de Fc_{ε}RI ou d'un homologue fonctionnellement équivalent; et
une molécule d'ADN recombinant codant pour un polypeptide ayant la séquence d'aminoacides d'une chaîne du dimère de la sous-unité γ de Fc_{ε}RI ou d'un homologue fonctionnellement équivalent,
et à cultiver ladite cellule transfectée dans des conditions où ledit complexe de polypeptide est produit par ladite cellule transfectée et à isoler ledit polypeptide.

11. Procédé d'obtention d'un complexe de polypeptide recombinant selon la revendication 10, dans lequel la molécule d'ADN recombinant codant pour la sous-unité a a une origine humaine et les molécules d'ADN recombinant codant pour la sous-unité β et la sous-unité γ proviennent chacune du rat.

12. Complexe de polypeptide recombinant ayant la séquence d'aminoacides du récepteur à forte affinité Fc_{ε}RI pour IgE ou d'un homologue fonctionnellement équivalent, susceptible d'être obtenu par le procédé de la revendication 11.

13. Procédé d'obtention d'un polypeptide synthétique ayant la séquence d'aminoacides d'une chaîne du dimère de la sous-unité γ ou d'un homologue fonctionnellement équivalent, dans lequel ladite séquence d'aminoacides est codée par un segment d'ADN selon l'une quelconque des revendications 1 à 6 et ledit procédé comprend l'utilisation d'un support en phase solide comme plate-forme de synthèse pour préparer ledit polypeptide synthétique.

14. Procédé d'obtention d'un complexe de polypeptide synthétique ayant la séquence d'aminoacides du récepteur à forte affinité Fc_{ε}RI pour IgE ou d'un homologue fonctionnellement équivalent, ledit complexe comprenant un dimère de la sous-unité α, de la sous-unité β et de la sous-unité γ, dans lequel la séquence d'aminoacides d'une chaîne dudit dimère de la sous-unité γ est codée par un segment d'ADN selon l'une quelconque des revendications 1 à 6, et ledit procédé comprend l'utilisation d'un support en phase solide comme plate-forme de synthèse pour préparer ledit polypeptide synthétique.
